# EUROPEAN PATENT APPLICATION

(11) **EP 4 685 143 A1**
(43) Date of publication of application: **28.01.2026**
(21) Application number: 24775184.5
(22) Date of filing: 20.03.2024
(51) Int. Cl.: C07D 413/04, A61K 31/4439, A61K 31/444, A61P 25/14, A61P 25/16, C07D 413/14, C07D 417/14

(54) **DOUBLE REGULATOR FOR MGLUR5 AND HDAC6 AND USE THEREOF**

(30) Priority: 21.03.2023 KR 20230036447
(71) Applicant: Vivozon,Inc., Giheung-gu Yongin-si, Gyeonggi-do 16914 (KR)
(72) Inventor: BAE, Mi Seon, Yongin-si, Gyeonggi-do 16914 (KR); LEE, Ju Hyeon, Yongin-si, Gyeonggi-do 16914 (KR); KIM, Hyo Jin, Yongin-si, Gyeonggi-do 16914 (KR); WOO, Seung A, Yongin-si, Gyeonggi-do 16914 (KR); HEO, Hyun Jin, Yongin-si, Gyeonggi-do 16914 (KR); NA, Ji Young, Yongin-si, Gyeonggi-do 16914 (KR); LEE, Geon Ho, Yongin-si, Gyeonggi-do 16914 (KR); CHOI, Dae Kyu, Yongin-si, Gyeonggi-do 16914 (KR)
(74) Representative: Vossius & Partner Patentanwälte Rechtsanwälte mbB
(86) International application number: PCT/KR2024/003472
(87) International publication number: WO 2024/196136

(57) **Abstract**

Disclosed are a double regulator for mGluR5 and HDAC6 and a use thereof. More specifically, disclosed are a compound that simultaneously regulates mGluR5 and HDAC6, and a use thereof as a therapeutic agent for movement disorders.

## Description

### TECHNICAL FIELD

Disclosed are a dual antagonist of mGluR5 and HDAC6, and use thereof. More specifically, disclosed are a compound which acts as an antagonist of mGluR5 and HDAC6 at the same time, and use thereof as a therapeutic agent for movement disorder.

### BACKGROUND ART

Movement disorders are a group of conditions that affect the ability to produce and control body movements and are often associated with neurological disorders or conditions associated with neurological dysfunction. Movement disorders may be shown as abnormal proficiency or speed of movements, excessive or involuntary movements, or slowness or absence of voluntary movements.

Movement disorders are frequently caused by impaired regulation of dopaminergic neurotransmission. Parkinson's disease (PD) is an example of a movement disorder associated with dysfunction in the regulation of dopaminergic neurotransmission caused by progressive degeneration of dopamine neurons. Tardive dyskinesia is another example of a movement disorder associated with dysfunction in the regulation of dopaminergic neurotransmission.

To replace lost dopamine, PD is currently treated with, for example, levodopa (L-DOPA, a precursor of dopamine). Unfortunately, the treatment of PD with L-DOPA often results in a specific form of dyskinesia called L-DOPA induced dyskinesia (LID), which is caused in part by excessive dopamine levels within the synapse.

Glutamate receptors are largely divided into ionotropic glutamate receptors (iGluR) and metabotropic glutamate receptors (mGluR). Among them, the metabotropic glutamate receptor is a type of G protein-coupled receptor (GPCR) and classified into three groups-I, II, and III, according to the characteristics of the signal transduction process. Group I consist of mGluR1 and mGluR5, and act as postsynaptic receptors involved in increasing neuronal excitability.

Histone deacetylases (HDACs) are enzymes that remove acetyl groups from lysine residues of histone proteins that make up chromatin. In humans, 18 HDACs are known and classified into Class I (HDAC1, 2, 3, 8), Class II (IIa: HDAC4, 5, 7, 9; IIb: HDAC6, 10), Class III (SIRT 1-7) and Class IV (HDAC11). HDAC6-which is one of the Class IIb HDACs-mainly exists in the cytoplasm and plays an important role in the microtubule network by regulating the balance of acetylation and deacetylation of tubulins in addition to histones.

### DISCLOSURE OF INVENTION

### TECHNICAL PROBLEM

An object of the present invention is to provide a compound that modulates mGluR5 and HDAC6 at the same time.

Another object of the present invention is to provide the use of said compound for the prevention or treatment of movement disorder.

### SOLUTION TO PROBLEM

To achieve the above object, there is provided a compound of the following Formula 1 or a pharmaceutically acceptable salt thereof: 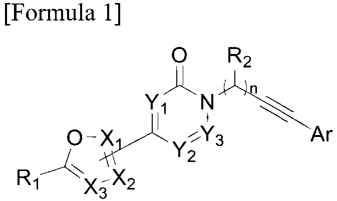 in Formula 1, Ar, R₁, R₂, X₁, X₂, X₃, Y₁, Y₂, Y₃ and n are the same as defined herein.

In addition, there is provided a pharmaceutical composition for the prevention or treatment of movement disorder comprising a therapeutically effective amount of the compound of Formula 1 or a pharmaceutically acceptable salt thereof as an active ingredient, together with a pharmaceutically acceptable carrier or excipient.

In addition, there is provided a method for treating movement disorder comprising administering to a mammal the compound of Formula 1 or a pharmaceutically acceptable salt thereof.

In addition, there is provided use of the compound of Formula 1 or a pharmaceutically acceptable salt thereof in the prevention or treatment of movement disorder.

### EFFECTS OF THE INVENTION

The compound of formula 1 or a pharmaceutically acceptable salt thereof according to the present invention acts as a dual antagonist of mGluR5 and HDAC6. Dual antagonism against mGluR5 and HDAC6 can have synergistic effects in preventing or treating movement disorders. Because of these properties, the compound of Formula 1 or a pharmaceutically acceptable salt thereof can exhibit preventive or therapeutic effects on movement disorders without specific side effects even at low doses.

### MODE FOR THE INVENTION

The present invention is described in detail hereinafter.

According to one aspect of the present invention, there is provided a compound of the following Formula 1 or a pharmaceutically acceptable salt thereof: 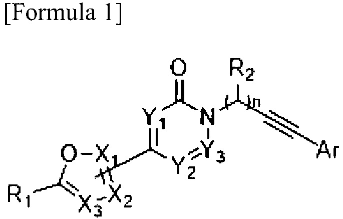 in Formula 1,
Ar is aryl or heteroaryl, wherein the aryl and heteroaryl are optionally substituted with one or more substituents selected from the group consisting of halo, cyano, alkyl, haloalkyl, alkoxy, haloalkoxy and alkylsulfonyl;
R₁ is alkyl or haloalkyl;
R₂ is H or alkyl;
one of X₁, X₂ and X₃ is C, and the others are N;
Y₁, Y₂ and Y₃ are each independently CR₃ or N; wherein R₃ is H, halo or alkyl;
n is an integer from 0 to 2; and
the heteroaryl has one or more heteroatoms selected from N, O and S.

Herein, the following concepts defined to the substituents are used to define the compound of Formula 1.

As used herein, the term "halo" or halogen," either alone or in combination with additional terms (for example, haloalkyl or haloalkoxy), refers to a radical of fluorine (F), chlorine (Cl), bromine (Br) or iodine (I).

As used herein, the term "cyano" refers to -CN.

As used herein, the term "sulfonyl" refers to -S(=O)₂-.

As used herein, the term "alkyl," either alone or in combination with additional terms (for example, haloalkyl), refers to a radical of a saturated aliphatic hydrocarbon group having, for example 1 to 7 carbon atoms of a linear or branched chain. The representative example of alkyl may include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl and the like, but is not limited thereto.

As used herein, the term "alkoxy" refers to alkyloxy (-O-alkyl) having, for example 1 to 7 carbon atoms.

As used herein, the term "aryl" refers to an aromatic hydrocarbon group having, for example 6 to 10 carbon atoms. For example, the aryl may include phenyl and naphthyl, but is not limited thereto.

As used herein, the term "heteroaryl" refers to, for example 5- to 10-membered aromatic hydrocarbons which includes one or more heteroatoms selected from N, O and S as a ring member.

According to one embodiment of the present invention, in the above Formula 1,
Ar is C₆-C₁₀ aryl or 5- to 10-membered heteroaryl, wherein the aryl and heteroaryl are optionally substituted with one or more substituents selected from the group consisting of halo, cyano, C₁-C₇ alkyl, halo-C₁-C₇ alkyl, C₁-C₇ alkoxy, halo-C₁-C₇ alkoxy and C₁-C₇ alkylsulfonyl;
R₁ is C₁-C₇ alkyl or halo-C₁-C₇ alkyl;
R₂ is H or C₁-C₇ alkyl;
one of X₁, X₂ and X₃ is C, and the others are N;
Y₁, Y₂ and Y₃ are each independently CR₃ or N; wherein R₃ is H, halo or C₁-C₇ alkyl;
n is an integer of 1 or 2; and
the heteroaryl has 1 to 3 heteroatoms selected from N, O and S.

According to another embodiment of the present invention, X₁ may be C, and X₂ and X₃ may be N. At this time, the above Formula 1 may be represented by the following Formula 2: 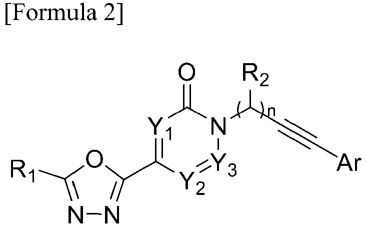 in Formula 2, Ar, R₁, R₂, Y₁, Y₂ and Y₃ are the same as defined in Formula 1, and n may be an integer of 0 to 2, specifically 1 or 2, and more specifically 1.

According to another embodiment of the present invention, X₂ may be C, and X₁ and X₃ may be N. At this time, the above Formula 1 may be represented by the following Formula 3: 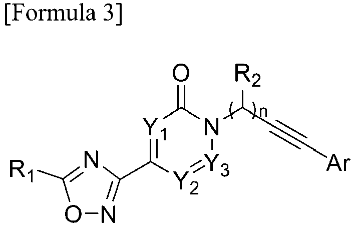 in Formula 3, Ar, R₁, R₂, Y₁, Y₂ and Y₃ are the same as defined in Formula 1, and n may be an integer of 0 to 2, specifically 1 or 2, and more specifically 1.

According to another embodiment of the present invention, the aryl may be phenyl; and the heteroaryl may be pyridyl, thienyl, imidazolyl, or benzothiazolyl.

According to another embodiment of the present invention, Ar may be phenyl or pyridyl. At this time, the above Formula 1 may be represented by the following Formula 4: 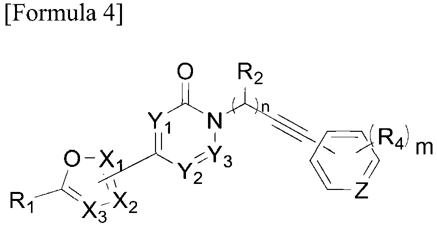 in Formula 4, Z is CH or N; R₄ is halo, cyano, alkyl, haloalkyl, alkoxy, haloalkoxy or alkylsulfonyl; m is an integer from 0 to 3; R₁, R₂, X₁, X₂, X₃, Y₁, Y₂, Y₃ and n are the same as defined in Formula 1.

According to another embodiment of the present invention, all of Y₁, Y₂ and Y₃ may be CR₃. According to another embodiment of the present invention, R₃ may be H.

In any one of Formulas 1 to 4, R₁ may be halo-C1 alkyl, and more specifically, R₁ may be CF₂H or CF₃.

According to another embodiment of the present invention, representative examples of the compound of Formula 1 may include the following compounds, but are not limited thereto:
4-(5-(difluoromethyl)-1,3,4-oxadiazol-2-yl)-1-(3-phenylprop-2-yn-1-yl)pyridin-2(1H)-one;
1-(3-phenylprop-2-yn-1-yl)-4-(5-(trifluoromethyl)-1,2,4-oxadiazol-3-yl)pyridin-2(1H)-one;
1-(3-(pyridin-4-yl)prop-2-yn-1-yl)-4-(5-(trifluoromethyl)-1,2,4-oxadiazol-3-yl)pyridin-2(1H)-one;
1-(3-(pyridin-3-yl)prop-2-yn-1-yl)-4-(5-(trifluoromethyl)-1,2,4-oxadiazol-3-yl)pyridin-2(1H)-one;
1-(3-(pyridin-2-yl)prop-2-yn-1-yl)-4-(5-(trifluoromethyl)-1,2,4-oxadiazol-3-yl)pyridin-2(1H)-one;
1-(3-(2-fluorophenyl)prop-2-yn-1-yl)-4-(5-(trifluoromethyl)-1,2,4-oxadiazol-3-yl)pyridin-2(1H)-one;
1-(3-(3-chlorophenyl)prop-2-yn-1-yl)-4-(5-(trifluoromethyl)-1,2,4-oxadiazol-3-yl)pyridine-2(1H)-one;
4-(5-(difluoromethyl)-1,3,4-oxadiazol-2-yl)-1-(3-(pyridin-4-yl)prop-2-yn-1-yl)pyridin-2(1H)-one;
4-(5-(difluoromethyl)-1,3,4-oxadiazol-2-yl)-1-(3-(pyridin-3-yl)prop-2-yn-1-yl)pyridin-2(1H)-one;
4-(5-(difluoromethyl)-1,3,4-oxadiazol-2-yl)-1-(3-(pyridin-2-yl)prop-2-yn-1-yl)pyridin-2(1H)-one;
4-(5-(difluoromethyl)-1,3,4-oxadiazol-2-yl)-1-(3-(4-fluorophenyl)prop-2-yn-1-yl)pyridin-2(1H)-one;
4-(5-(difluoromethyl)-1,3,4-oxadiazol-2-yl)-1-(3-(3-fluorophenyl)prop-2-yn-1-yl)pyridin-2(1H)-one;
4-(5-(difluoromethyl)-1,3,4-oxadiazol-2-yl)-1-(3-(2-fluorophenyl)prop-2-yn-1-yl)pyridin-2(1H)-one;
1-(3-(4-chlorophenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(difluoromethyl)-1,3,4-oxadiazol-2-yl)pyridin-2(1H)-one;
1-(3-(2-chlorophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(p-tolyl)prop-2-yn-1-yl)pyridin-2(1H)-one;
4-(5-(difluoromethyl)-1,3,4-oxadiazol-2-yl)-1-(3-(m-tolyl)prop-2-yn-1-yl)pyridin-2(1H)-one;
4-(5-(difluoromethyl)-1,3,4-oxadiazol-2-yl)-1-(3-(3-(trifluoromethyl)phenyl)prop-2-yn-1-yl)pyridin-2(1H)-one;
4-(5-(difluoromethyl)-1,3,4-oxadiazol-2-yl)-1-(3-(3-(trifluoromethoxy)phenyl)prop-2-yn-1-yl)pyridin-2(1H)-one;
4-(5-(difluoromethyl)-1,3,4-oxadiazol-2-yl)-1-(3-(3-methoxyphenyl)prop-2-yn-1-yl)pyridin-2(1H)-one;
1-(3-(6-chloropyridin-2-yl)prop-2-yn-1-yl)-4-(5-(difluoromethyl)-1,3,4-oxadiazol-2-yl)pyridin-2(1H)-one;
1-(3-(2-chloropyridin-4-yl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(6-methylpyridin-2-yl)prop-2-yn-1-yl)pyridin-2(1H)-one;
1-(3-(3-bromophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(3-(difluoromethyl)phenyl)prop-2-yn-1-yl)pyridin-2(1H)-one;
1-(3-(3-(difluoromethoxy)phenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(trifluoromethyl)-1,3,4-oxadiazol-2-yl)pyridin-2(1H)-one;
4-(5-(difluoromethyl)-1,3,4-oxadiazol-2-yl)-1-(3-(5-methylpyridin-3-yl)prop-2-yn-1-yl)pyridin-2(1H)-one;
1-(3-(3-chlorophenyl)prop-2-yn-1-yl)-4-(5-(difluoromethyl)-1,2,4-oxadiazol-3-yl)pyridin-2(1H)-one;
3-(3-(4-(5-(difluoromethyl)-1,3,4-oxadiazol-2-yl)-2-oxopyridin-1(2H)-yl)prop-1-yn-1-yl)benzonitrile;
4-(5-(difluoromethyl)-1,3,4-oxadiazol-2-yl)-1-(3-(3-(methylsulfonyl)phenyl)prop-2-yn-1-yl)pyridin-2(1H)-one;
1-(3-(3-chloro-2-fluorophenyl)prop-2-yn-1-yl)-4-(5-(difluoromethyl)-1,3,4-oxadiazol-2-yl)pyridin-2(1H)-one;
1-(3-(3-chloro-4-fluorophenyl)prop-2-yn-1-yl)-4-(5-(difluoromethyl)-1,3,4-oxadiazol-2-yl)pyridin-2(1H)-one;
1-(3-(3-chloro-5-fluorophenyl)prop-2-yn-1-yl)-4-(5-(difluoromethyl)-1,3,4-oxadiazol-2-yl)pyridin-2(1H)-one;
1-(3-(5-chloro-2-fluorophenyl)prop-2-yn-1-yl)-4-(5-(difluoromethyl)-1,3,4-oxadiazol-2-yl)pyridin-2(1H)-one;
2-(3-(3-chlorophenyl)prop-2-yn-1-yl)-5-(5-(difluoromethyl)-1,3,4-oxadiazol-2-yl)pyridazin-3(2H)-one;
1-(3-(3-chlorophenyl)prop-2-yn-1-yl)-4-(5-(difluoromethyl)-1,3,4-oxadiazol-2-yl)pyrimidin-2(1H)-one;
3-(3-(3-chlorophenyl)prop-2-yn-1-yl)-6-(5-(difluoromethyl)-1,3,4-oxadiazol-2-yl)pyrimidin-4(3H)-one;
1-(3-(benzo]thiazol-5-yl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(2-methylbenzo[d]thiazol-6-yl)prop-2-yn-1-yl)pyridin-2(1H)-one;
4-(5-(difluoromethyl)-1,3,4-oxadiazol-2-yl)-1-(3-(2-methyl-1H-imidazol-5-yl)prop-2-yn-1-yl)pyridin-2(1H)-one;
4-(5-(difluoromethyl)-1,3,4-oxadiazol-2-yl)-1-(3-(thiophen-3-yl)prop-2-yn-1-yl)pyridin-2(1H)-one;
1-(1-(3-chlorophenyl)pent-1-yn-3-yl)-4-(5-(difluoromethyl)-1,3,4-oxadiazol-2-yl)pyridin-2(1H)-one;
1-(3-(3-chlorophenyl)prop-2-yn-1-yl)-4-(5-(difluoromethyl)-1,3,4-oxadiazol-2-yl)-6-methylpyridin-2(1H)-one; and
5-bromo-1-(3-(3-chlorophenyl)prop-2-yn-1-yl)-4-(5-(difluoromethyl)-1,3,4-oxadiazol-2-yl)pyridin-2(1H)-one.

According to another embodiment of the present invention, more specific examples of the compound of Formula 1 may include the following compounds:
1-(3-(3-chlorophenyl)prop-2-yn-1-yl)-4-(5-(trifluoromethyl)-1,2,4-oxadiazol-3-yl)pyridin-2(1H)-one;
1-(3-(3-chlorophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(p-tolyl)prop-2-yn-1-yl)pyridin-2(1H)-one;
4-(5-(difluoromethyl)-1,3,4-oxadiazol-2-yl)-1-(3-(m-tolyl)prop-2-yn-1-yl)pyridin-2(1H)-one;
4-(5-(difluoromethyl)-1,3,4-oxadiazol-2-yl)-1-(3-(3-(trifluoromethyl)phenyl)prop-2-yn-1-yl)pyridin-2(1H)-one;
4-(5-(difluoromethyl)-1,3,4-oxadiazol-2-yl)-1-(3-(3-(trifluoromethoxy)phenyl)prop-2-yn-1-yl)pyridin-2(1H)-one;
1-(3-(2-chloropyridin-4-yl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(6-methylpyridin-2-yl)prop-2-yn-1-yl)pyridin-2(1H)-one;
1-(3-(3-bromophenyl)prop-2-yn-1-yl)-4-(5-(difluoromethyl)-1,3,4-oxadiazol-2-yl)pyridin-2(1H)-one;
1-(3-(3-(difluoromethoxy)phenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(trifluoromethyl)-1,3,4-oxadiazol-2-yl)pyridin-2(1H)-one;
1-(3-(3-chlorophenyl)prop-2-yn-1-yl)-4-(5-(difluoromethyl)-1,2,4-oxadiazol-3-yl)pyridin-2(1H)-one;
3-(3-(4-(5-(difluoromethyl)-1,3,4-oxadiazol-2-yl)-2-oxopyridin-1(2H)-yl)prop-1-yn-1-yl)benzonitrile;
1-(3-(5-chloro-2-fluorophenyl)prop-2-yn-1-yl)-4-(5-(difluoromethyl)-1,3,4-oxadiazol-2-yl)pyridin-2(1H)-one;
1-(1-(3-chlorophenyl)pent-1-yn-3-yl)-4-(5-(difluoromethyl)-1,3,4-oxadiazol-2-yl)pyridin-2(1H)-one; and
1-(3-(3-chlorophenyl)prop-2-yn-1-yl)-4-(5-(difluoromethyl)-1,3,4-oxadiazol-2-yl)-6-methylpyridin-2(1H)-one.

### Preparation Methods

The compound of Formula 1 according to one embodiment can be prepared by anyone with ordinary knowledge of compound synthesis in this technical field using known compounds or compounds that can be easily prepared therefrom. For example, the compound of Formula 1 can be synthesized according to the methods of Reaction Schemes 1 to 3 below, but this merely presents one exemplary method and the order of unit operations can be selectively changed as needed. It is not intended to limit the scope of the invention.

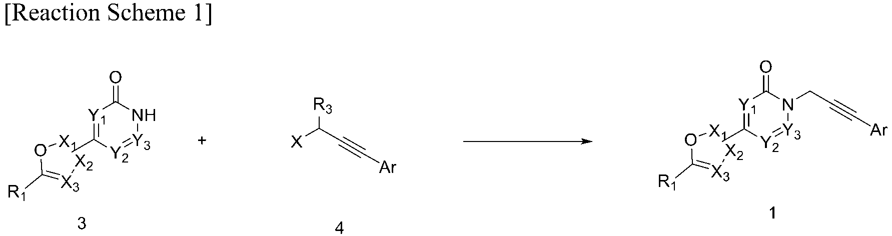

In Reaction Scheme 1, X is a leaving group such as mesylate (MsO). Intermediate 3 can be prepared, for example, as shown in Reaction Scheme 1' below.

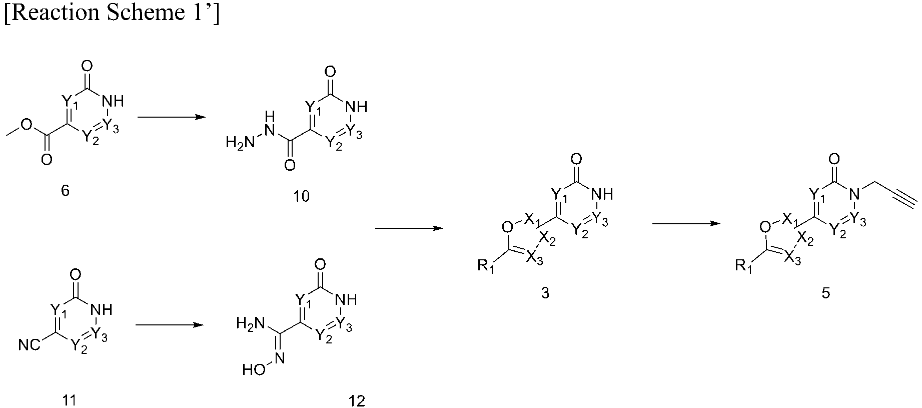

Intermediate 10 or 12 is obtained from each starting material containing ester or cyano by adding hydrazine or hydroxylamine, and then Intermediate 3 is obtained through a cyclization reaction by adding trifluoroacetic anhydride. If necessary, Intermediate 5 containing acetylene may also be obtained by additional nucleophilic addition reaction.

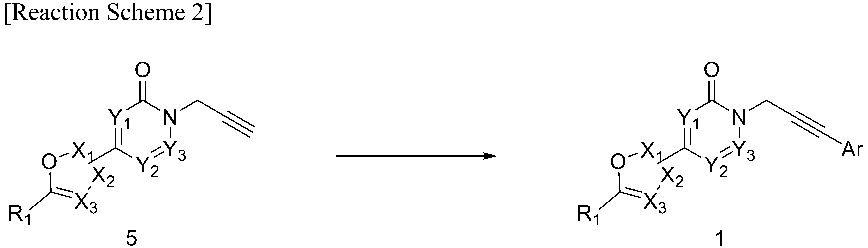

In Reaction Scheme 2, Compound 1 may be obtained from acetylene Intermediate 5 and a halogen-containing reagent through Sonogashira coupling. Intermediate 5 may be prepared by various methods including Scheme 1' above or Scheme 2' below.

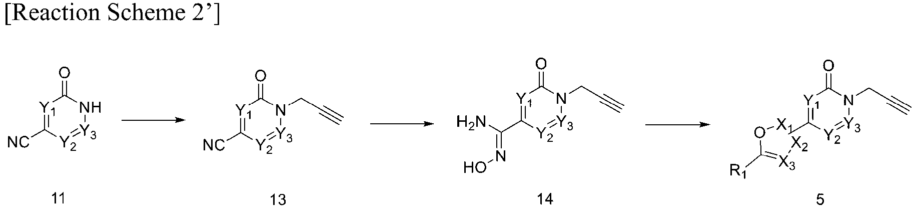

Intermediate 13 containing acetylene is obtained from starting material 11 containing cyano through a nucleophilic addition reaction, and then hydroxylamine is added to obtain Intermediate 14. Then, Intermediate 5 may be obtained through a cyclization reaction with the addition of trifluoroacetic anhydride.

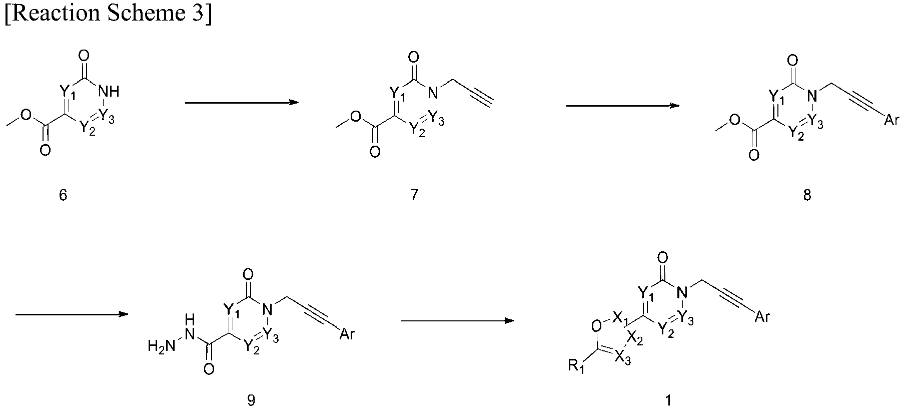

Intermediate 7 containing acetylene is obtained from starting material 6 containing ester through a nucleophilic addition reaction, and Intermediate 8 is obtained through Sonogashira coupling. Consequently, Intermediate compound No. 9 is obtained by adding hydrazine, and then Compound 1 may be obtained through a cyclization reaction by adding trifluoroacetic anhydride.

### Pharmaceutical Composition

According to another aspect of the present invention, there is provided a pharmaceutical composition for the prevention or treatment of movement disorder comprising a therapeutically effective amount of the compound of Formula 1 or pharmaceutically acceptable salt thereof as active ingredient, together with a pharmaceutically acceptable carrier or excipient.

The pharmaceutical composition may be formulated in various oral or parenteral dosage forms. For example, the pharmaceutical composition may be formulated into any dosage form for oral administration, such as tablets, pills, hard/soft capsules, solutions, suspensions, emulsifiers, syrups, granules or elixirs.

When the pharmaceutical composition is formulated into a parenteral dosage form, the pharmaceutical composition may be administered by a parenteral administration method such as subcutaneous injection, intravenous injection, intramuscular injection or intrathoracic injection. The pharmaceutical composition may be prepared as a solution or a suspension by mixing an active ingredient-i.e., the compound of Formula 1 or a pharmaceutically acceptable salt thereof, with a stabilizer or a buffer in water, and the solution or the suspension may be prepared as a unit dosage form of an ampoule or a vial.

### Medical Usefulness

According to still another aspect of the present invention, there is provided a method for preventing or treating movement disorder in a subject, comprising administering to the subject in need thereof a therapeutically effective amount of the compound of Formula 1 or a pharmaceutically acceptable salt thereof. The movement disorder may include, for example, Huntington's disease, Parkinson's disease and levodopa-induced dyskinesia (LID), but is not limited thereto.

Symptoms of Parkinson's disease appear when the ionotropic glutamate (iGlu) receptors and metabotropic glutamate (mGlu) receptors of glutamate-which is an excitatory brain transmitter-are activated. Among them, metabotropic glutamate receptor 5 (mGluR5) is mainly expressed in postsynaptic cell membranes (Shigemoto et al., 1997), and is also found in presynaptic cell membranes and glial cells. Therefore, in Parkinson's disease and levodopa-induced complications (PD-LID), pharmacological inhibition of metabotropic glutamate receptor 5 (mGluR5) regulates postsynaptic excitatory synaptic transmission and glutamatergic hyperactivity, thereby showing potential as a therapeutic target (Huber et al., 2002).

The mechanism of HDAC6 (histone deacetylase 6) is unclear, but it is known to have a brain-protective effect in neurological diseases such as Huntington's disease and Parkinson's disease. In general, HDAC6 is believed to affect the development of neurological diseases through various pathways such as forming aggresomes, increasing autophagy and removing misfolded proteins (de Zoeten et al., 2011, d'Ydewalle et al., 2011, Fukuda et al., 2012, Govindarajan et al., 2013).

Changes in cytoprotective activity are associated with various neurodegenerative pathologies, such as Parkinson's disease (Outerio et al., Science (2007), (5837), 516-519) and Huntington's disease (Dompierre et al., J. Neurosci. (2007), 27(13), 3571-3583), and it is known that inhibition of HDAC6 has a protective effect and prevents the death of α-synuclein neurons. At the same time, it can inhibit the aggregation of α-synuclein and protect nerve cells by increasing autophagy activity, which is involved in the regulation of α-synuclein accumulation.

The compound of Formula 1 acts as a dual antagonist of mGluR5 and HDAC. These compounds can have effects on reducing excitatory neuron damage and inhibiting microglial activity through inhibition of metabotropic glutamate receptors (mGluR5) among the various mechanisms of Parkinson's disease, and have neuroprotective effects through inhibition of HDAC6. Therefore, the compound of Formula 1 can be effectively used as a pharmaceutical composition for treating Parkinson's disease and suppressing levodopa-induced complications.

In another embodiment according to the present invention, a method for preventing or treating movement disorder comprises administering to an animal a pharmaceutical composition comprising an effective amount of the compound of Formula 1 and a pharmaceutically acceptable carrier or excipient. The method is particularly suitable for use in humans, but may also be used in other animals, particularly mammals.

The specific administration method and therapeutically effective amount of the compound of Formula 1 or a pharmaceutically acceptable salt thereof may be determined by a person skilled in the art in consideration of the type of target mammal, the type of disease, and the type of the compound of Formula 1, and not specifically limited.

For example, the compound of Formula 1 or a pharmaceutically acceptable salt thereof may be included in the pharmaceutical composition in an effective dose of 0.1 to 1,000 mg/kg (body weight), preferably 0.5 to 500 mg/kg (body weight) per day for mammals including humans. The pharmaceutical composition may be administered once or divided two or more times a day and administered through an oral or parenteral route.

### Examples

Hereinafter, the present invention is explained in more detail with the following examples. However, it must be understood that the protection scope of the present invention is not limited to the examples.

### Example 1: Synthesis of 4-(5-(difluoromethyl)-1,3,4-oxadiazol-2-yl)-1-(3-phenylprop-2-yn-1-yl)pyridin-2(1H)-one

### Step 1: Synthesis of methyl 2-oxo-1-(prop-2-yn-1-yl)-1,2-dihydropyridine-4-carboxylate

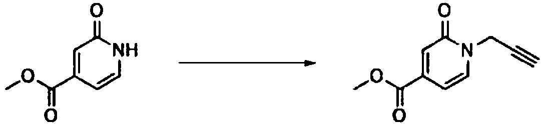

Methyl 2-oxo-1,2-dihydropyridine-4-carboxylate (1.000 g, 6.530 mmol), 3-bromoprop-1-yne (80.00% solution in toluene, 0.618 mL, 6.530 mmol) and potassium carbonate (1.805 g, 13.060 mmol) were dissolved in *N*,*N*-dimethylformamide (30 mL) at room temperature and stirred at the same temperature for 18 hours. Water was poured into the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain methyl 2-oxo-1-(prop-2-yn-1-yl)-1,2-dihydropyridine-4-carboxylate (0.720 g, 57.7%) in the form of a white solid.

### Step 2: Synthesis of methyl 2-oxo-1-(3-phenylprop-2-yn-1-yl)-1,2-dihydropyridine-4-carboxylate

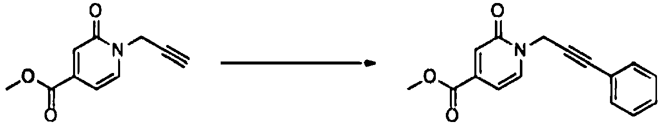

Methyl 2-oxo-1-(prop-2-yn-1-yl)-1,2-dihydropyridine-4-carboxylate (0.720 g, 3.766 mmol) and triethylamine (2.624 mL, 18.829 mmol) were dissolved in tetrahydrofuran (10 mL) at room temperature, and bis(triphenylphosphine)palladium(II) dichloride (0.132 g, 0.188 mmol) and copper iodide (CuI, 0.086 g, 0.452 mmol) were added thereto and stirred at the same temperature for 10 minutes. Iodobenzene (0.462 mL, 4.142 mmol) was added to the reaction mixture and stirred at the same temperature for additional 18 hours. The reaction mixture was filtered through a Celite pad to remove solids, the filtrate was treated under reduced pressure to remove the solvent, and the concentrate was purified and concentrated by column chromatography to obtain methyl 2-oxo-1-(3-phenylprop-2-yn-1-yl)-1,2-dihydropyridine-4-carboxylate (0.658 g, 65.4%) in the form of a yellow solid.

### Step 3: Synthesis of 2-oxo-1-(3-phenylprop-2-yn-1-yl)-1,2-dihydropyridine-4-carbohydrazide

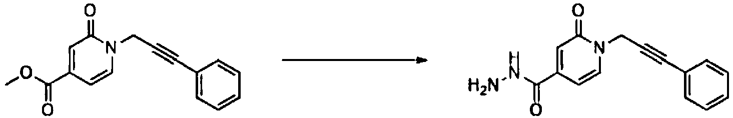

Methyl 2-oxo-1-(3-phenylprop-2-yn-1-yl)-1,2-dihydropyridine-4-carboxylate (0.658 g, 2.462 mmol) and hydrazine monohydrate (1.197 mL, 24.618 mmol) were dissolved in ethanol (20 mL) at room temperature was stirred at 80°C for 5 hours, and then the temperature was lowered to room temperature to terminate the reaction. Water was poured into the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 2-oxo-1-(3-phenylprop-2-yn-1-yl)-1,2-dihydropyridine-4-carbohydrazide (0.414 g, 62.9%) in the form of a yellow solid: **LRMS** (ES) *m*/*z* 268.06 [M+H]⁺, calculated MW 267.29.

### Step 4: Synthesis of 4-(5-(difluoromethyl)-1,3,4-oxadiazol-2-yl)-1-(3-phenylprop-2-yn-1-yl)pyridin-2(1H)-one

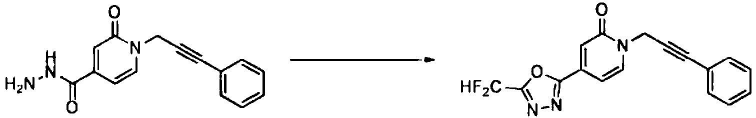

2-Oxo-1-(3-phenylprop-2-yn-1-yl)-1,2-dihydropyridine-4-carbohydrazide (0.414 g, 1.549 mmol), 2,2-difluoroacetic anhydride (0.578 mL, 4.647 mmol) and triethylamine (0.648 mL, 4.647 mmol) were mixed with tetrahydrofuran (10 mL) at room temperature. The obtained mixture was heated to reflux for 1 hour and then lowered to room temperature, and a saturated sodium bicarbonate aqueous solution was added to the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 4-(5-(difluoromethyl)-1,3,4-oxadiazol-2-yl)-1-(3-phenylprop-2-yn-1-yl)pyridin-2(1H)-one (0.300 g, 59.2%) in the form of a white solid: **LRMS** (ES) *m*/*z* 328.11 [M+H]⁺, calculated MW 327.29; **¹H-NMR** (400 MHz, CD₃OD) d 8.09 (d, *J* = 7.6 Hz, 1 H), 7.44 (dd, *J* = 7.8, 1.8 Hz, 2 H), 7.35 ~ 7.31 (m, 3 H), 7.22 (t, *J* = 51.8 Hz, 1 H), 7.21 (d, *J* = 1.6 Hz, 1 H), 7.03 (dd, *J* = 7.4, 1.8 Hz, 1 H), 5.06 (s, 2 H).

### Example 2: Synthesis of 1-(3-phenylprop-2-yn-1-yl)-4-(5-(trifluoromethyl)-1,2,4-oxadiazol-3-yl)pyridin-2(1H)-one

### Step 1: Synthesis of 2-oxo-1-(prop-2-yn-1-yl)-1,2-dihydropyridine-4-carbonitrile

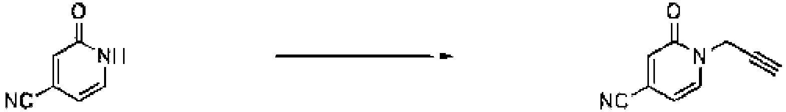

2-Oxo-1,2-dihydropyridine-4-carbonitrile (3.000 g, 24.977 mmol), 3-bromoprop-1-yne (80.00 % solution in toluene, 2.366 mL, 24.977 mmol) and potassium carbonate (6.904 g, 49.954 mmol) dissolved in *N*,*N*-dimethylformamide (30 mL) at room temperature and stirred at the same temperature for 18 hours. Water was poured into the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 2-oxo-1-(prop-2-yn-1-yl)-1,2-dihydropyridine-4-carbonitrile (3.000 g, 75.9%) in the form of an ivory solid.

### Step 2: Synthesis of N'-hydroxy-2-oxo-1-(prop-2-yn-1-yl)-1,2-dihydropyridine-4-carboximidamide

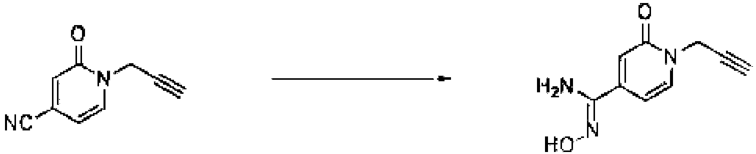

2-Oxo-1-(prop-2-yn-1-yl)-1,2-dihydropyridine-4-carbonitrile (3.000 g, 18.968 mmol), hydroxylamine (50.00% solution, 6.265 mL, 94.841 mmol) and triethylamine (13.219 mL, 94.841 mmol) were mixed with ethanol (100 mL) at room temperature. The obtained mixture was heated to reflux for 2 hours and then lowered to room temperature, and water was poured into the reaction mixture and extracted with dichloromethane. The organic layer was washed with saturated aqueous sodium chloride solution, dried over anhydrous sodium sulfate, filtered and concentrated under reduced pressure. The precipitated solid was filtered, washed with ethanol and dried to obtain *N'*-hydroxy-2-oxo-1-(prop-2-yn-1-yl)-1,2-dihydropyridine-4-carboximidamide (2.250 g, 62.0%) in the form of a white solid.

### Step 3: Synthesis of 1-(prop-2-yn-1-yl)-4-(5-(trifluoromethyl)-1,2,4-oxadiazol-3-yl)pyridin-2(1H)-one

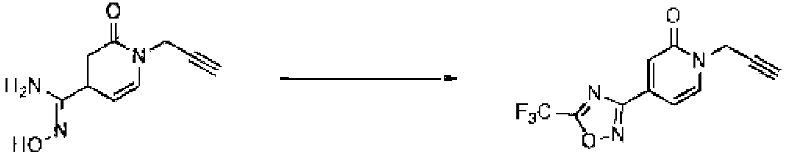

*N'*-hydroxy-2-oxo-1-(prop-2-yn-1-yl)-1,2-dihydropyridine-4-carboximidamide (2.250 g, 11.768 mmol), trifluoroacetic anhydride (2.493 mL, 17.653 mmol) and triethylamine (2.460 mL, 17.653 mmol) were mixed with tetrahydrofuran (50 mL) at room temperature. The obtained mixture was heated to reflux for 1 hour and then lowered to room temperature, and a saturated sodium bicarbonate aqueous solution was added to the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 1-(prop-2-yn-1-yl)-4-(5-(trifluoromethyl)-1,2,4-oxadiazol-3-yl)pyridin-2(1*H*)-one (2.000 g, 63.1%) in the form of an ivory solid.

### Step 4: Synthesis of 1-(3-phenylprop-2-yn-1-yl)-4-(5-(trifluoromethyl)-1,2,4-oxadiazol-3-yl)pyridin-2(1H)-one

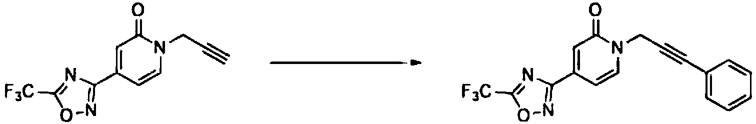

1-(Prop-2-yn-1-yl)-4-(5-(trifluoromethyl)-1,2,4-oxadiazol-3-yl)pyridin-2(1*H*)-one (0.100 g, 0.371 mmol), triethylamine (0.259 mL, 1.857 mmol), bis(triphenylphosphine)palladium(II) dichloride (0.013 g, 0.019 mmol) and copper iodide (CuI, 0.008 g, 0.045 mmol) were dissolved in tetrahydrofuran (5 mL) and stirred at room temperature for 10 minutes, and iodobenzene (0.046 mL, 0.409 mmol) was added thereto and stirred at the same temperature for additional 18 hours. The reaction mixture was filtered through a Celite pad to remove solids, and water was added to the filtrate and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 1-(3-phenylprop-2-yn-1-yl)-4-(5-(trifluoromethyl)-1,2,4-oxadiazol-3-yl)pyridin-2(1H)-one (0.065 g, 50.7%) in the form of a white solid: **LRMS** (ES) *m*/*z* 346.04 [M+H]⁺, calculated MW 345.28; **¹H-NMR** (400 MHz, CD₃OD) d 8.07 (d, *J* = 6.8 Hz, 1 H), 7.45 ~ 7.43 (m, 2 H), 7.35 ~ 7.28 (m, 4 H), 7.03 (dd, *J* = 7.2, 2.0 Hz, 1 H), 5.06 (s, 2 H).

### Example 3: Synthesis of 1-(3-(pyridin-4-yl)prop-2-yn-1-yl)-4-(5-(trifluoromethyl)-1,2,4-oxadiazol-3-yl)pyridin-2(1H)-one

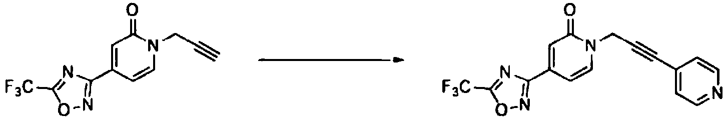

1-(Prop-2-yn-1-yl)-4-(5-(trifluoromethyl)-1,2,4-oxadiazol-3-yl)pyridin-2(1*H*)-one (0.100 g, 0.371 mmol), triethylamine (0.259 mL, 1.857 mmol), bis(triphenylphosphine)palladium(II) dichloride (0.013 g, 0.019 mmol), copper iodide (CuI, 0.008 g, 0.045 mmol) and 4-iodopyridine (0.084 g, 0.409 mmol) as starting materials were used in a similar manner to Step 4 of Example 2 to obtain 1-(3-(pyridin-4-yl)prop-2-yn-1-yl)-4-(5-(trifluoromethyl)-1,2,4-oxadiazol-3-yl)pyridin-2(1*H*)-one (0.059 g, 45.9%) in the form of a yellow solid: **LRMS** (ES) *m*/*z* 347.02 [M+H]⁺, calculated MW 346.27; **¹H-NMR** (400 MHz, CD₃OD) d 8.51 (d, *J* = 4.0 Hz, 2 H), 8.03 (d, *J* = 7.2 Hz, 1 H), 7.44 (d, *J* = 6.0 Hz, 2 H), 7.28 (d, *J* = 2.0 Hz, 1 H), 7.01 (dd, *J* = 7.0, 2.2 Hz, 1 H), 5.12 (s, 2 H).

### Example 4: Synthesis of 1-(3-(pyridin-3-yl)prop-2-yn-1-yl)-4-(5-(trifluoromethyl)-1,2,4-oxadiazol-3-yl)pyridin-2(1H)-one

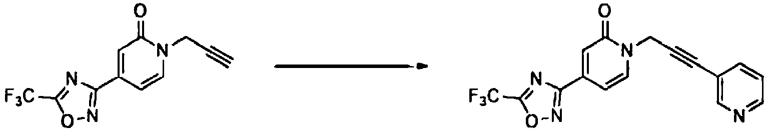

1-(Prop-2-yn-1-yl)-4-(5-(trifluoromethyl)-1,2,4-oxadiazol-3-yl)pyridin-2(1H)-one (0.100 g, 0.371 mmol), triethylamine (0.259 mL, 1.857 mmol), bis(triphenylphosphine)palladium(II) dichloride (0.013 g, 0.019 mmol), copper iodide (CuI, 0.008 g, 0.045 mmol) and 3-iodopyridine (0.084 g, 0.409 mmol) as starting materials were used in a similar manner to Step 4 of Example 2 to obtain 1-(3-(pyridin-3-yl)prop-2-yn-1-yl)-4-(5-(trifluoromethyl)-1,2,4-oxadiazol-3-yl)pyridin-2(1H)-one (0.030 g, 23.3%) in the form of a yellow solid: **LRMS** (ES) *m*/*z* 347.13 [M+H]⁺, calculated MW 346.27; **¹H-NMR** (400 MHz, CD₃OD) d 8.62 (d, *J* = 1.2 Hz, 1 H), 8.49 (dd, *J* = 4.6, 1.4 Hz, 1 H), 8.06 (d, *J* = 7.2 Hz, 1 H), 7.90 (dt, *J* = 7.6, 2.0 Hz, 1 H), 7.41 (dd, *J* = 7.8, 5.0 Hz, 1 H), 7.28 (d, *J* = 1.2 Hz, 1 H), 7.02 (dd, *J* = 7.0, 2.2 Hz, 1 H), 5.12 (s, 2 H).

### Example 5: Synthesis of 1-(3-(pyridin-2-yl)prop-2-yn-1-yl)-4-(5-(trifluoromethyl)-1,2,4-oxadiazol-3-yl)pyridin-2(1H)-one

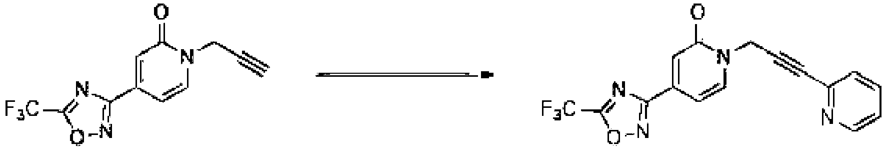

1-(Prop-2-yn-1-yl)-4-(5-(trifluoromethyl)-1,2,4-oxadiazol-3-yl)pyridin-2(1*H*)-one (0.100 g, 0.371 mmol), triethylamine (0.259 mL, 1.857 mmol), bis(triphenylphosphine)palladium(II) dichloride (0.013 g, 0.019 mmol), copper iodide (CuI, 0.008 g, 0.045 mmol) and 2-iodopyridine (0.084 g, 0.409 mmol) as starting materials were used in a similar manner to Step 4 of Example 2 to obtain 1-(3-(pyridin-2-yl)prop-2-yn-1-yl)-4-(5-(trifluoromethyl)-1,2,4-oxadiazol-3-yl)pyridin-2(1*H*)-one (0.080 g, 62.2%) in the form of a yellow solid: **LRMS** (ES) *m*/*z* 347.02 [M+H]⁺, calculated MW 346.27; **¹H-NMR** (400 MHz, CD₃OD) d 8.09 (d, *J* = 6.8 Hz, 1 H), 7.83 (t, *J* = 7.0 Hz, 1 H), 7.65 ~ 7.53 (m, 2 H), 7.41 (t, *J* = 6.0 Hz, 1 H), 7.28 (d, *J* = 1.2 Hz, 1 H), 7.02 (dd, *J* = 7.2, 1.6 Hz, 1 H), 5.13 (s, 2 H).

### Example 6: Synthesis of 1-(3-(2-fluorophenyl)prop-2-yn-1-yl)-4-(5-(trifluoromethyl)-1,2,4-oxadiazol-3-yl)pyridin-2(1H)-one

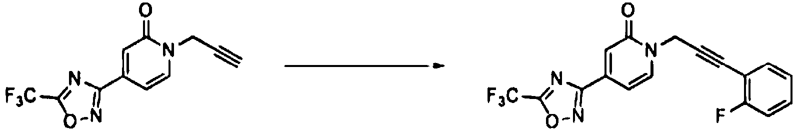

1-(Prop-2-yn-l-yl)-4-(5-(trifluoromethyl)-1 ,2,4-oxadiazol-3-yl)pyridin-2(1*H*)-one (0.100 g, 0.371 mmol), triethylamine (0.259 mL, 1.857 mmol), bis(triphenylphosphine)palladium(II) dichloride (0.013 g, 0.019 mmol), copper iodide (CuI, 0.008 g, 0.045 mmol) and 1-fluoro-2-iodobenzene (0.091 g, 0.409 mmol) as starting materials were used in a similar manner to Step 4 of Example 2 to obtain 1-(3-(2-fluorophenyl)prop-2-yn-1-yl)-4-(5-(trifluoromethyl)-1,2,4-oxadiazol-3-yl)pyridin-2(1*H*)-one (0.045 g, 33.3%) in the form of a white solid: **LRMS** (ES) m/z 364.03 [M+H]⁺, calculated MW 363.27; **¹H-NMR** (400 MHz, CD₃OD) d 8.07 (d, *J* = 6.8 Hz, 1 H), 7.47 (td, *J* = 7.5, 1.7 Hz, 1 H), 7.41 ~ 7.35 (m, 1 H), 7.29 (d, *J* = 1.6 Hz, 1 H), 7.16 ~ 7.10 (m, 2 H), 7.03 (dd, *J* = 7.2, 1.6 Hz, 1 H), 5.12 (s, 2 H).

### Example 7: Synthesis of 1-(3-(3-chlorophenyl)prop-2-yn-1-yl)-4-(5-(trifluoromethyl)-1,2,4-oxadiazol-3-yl)pyridine-2(1H)-one

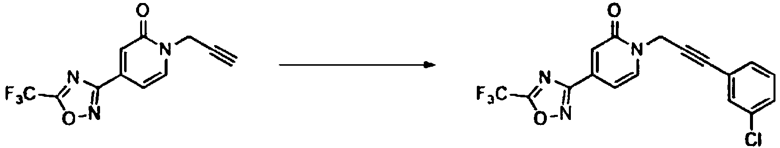

1-(Prop-2-yn-1-yl)-4-(5-(trifluoromethyl)-1,2,4-oxadiazol-3-yl)pyridin-2(1*H*)-one (0.100 g, 0.371 mmol), triethylamine (0.259 mL, 1.857 mmol), bis(triphenylphosphine)palladium(II) dichloride (0.013 g, 0.019 mmol), copper iodide (CuI, 0.008 g, 0.045 mmol) and 1-chloro-3-iodobenzene (0.097 g, 0.409 mmol) as starting materials were used in a similar manner to Step 4 of Example 2 to obtain 1-(3-(3-chlorophenyl)prop-2-yn-1-yl)-4-(5-(trifluoromethyl)-1,2,4-oxadiazol-3-yl)pyridin-2(1H)-one (0.082 g, 58.1%) in the form of a white solid: **LRMS** (ES) *m*/*z* 380.00 [M+H]⁺, calculated MW 379.72; **¹H-NMR** (400 MHz, CD₃OD) d 8.05 (d, *J* = 6.8 Hz, 1 H), 7.46 (s, 1 H), 7.38 (dd, *J* = 2.6, 1.4 Hz, 1 H), 7.36 (dd, *J* = 3.2, 1.2 Hz, 1 H), 7.32 (d, *J* = 7.6 Hz, 1 H), 7.28 (d, *J* = 2.0 Hz, 1 H), 7.02 (dd, *J* = 7.4, 1.8 Hz, 1 H), 5.07 (s, 2 H).

### Example 8: Synthesis of 4-(5-(difluoromethyl)-1,3,4-oxadiazol-2-yl)-1-(3-(pyridin-4-yl)prop-2-yn-1-yl)pyridin-2(1H)-one

### Step 1: Synthesis of 2-oxo-1,2-dihydropyridine-4-carbohydrazide

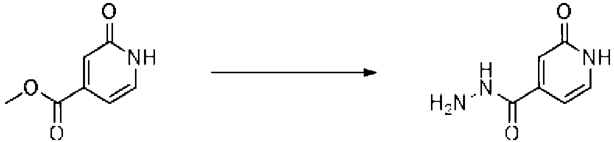

Methyl 2-oxo-1,2-dihydropyridine-4-carboxylate (3.000 g, 19.590 mmol) and hydrazine monohydrate (1.143 mL, 23.508 mmol) were dissolved in ethanol (100 mL) at room temperature. The obtained solution was stirred at 80°C for 18 hours, and the temperature was lowered to room temperature to terminate the reaction. The precipitated solid was filtered and dried to obtain 2-oxo-1,2-dihydropyridine-4-carbohydrazide (2.610 g, 87.0%) in the form of an ivory solid: **LRMS** (ES) *m*/*z* 153.98 [M+*H*]⁺, calculated MW 153.14.

### Step 2: Synthesis of 4-(5-(difluoromethyl)-1,3,4-oxadiazol-2-yl)pyridin-2(1H)-one

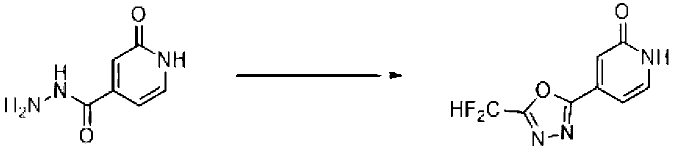

2-Oxo-1,2-dihydropyridine-4-carbohydrazide (2.610 g, 17.043 mmol), 2,2-difluoroacetic anhydride (6.357 mL, 51.130 mmol) and imidazole (3.481 g, 51.130 mmol) mmol) were dissolved in dichloromethane (30 mL) at room temperature. The obtained solution was heated and refluxed for 18 hours, and then the temperature was lowered to room temperature to terminate the reaction. The reaction mixture was filtered through a paper filter to remove solids, a saturated sodium bicarbonate aqueous solution was poured into the filtrate and extracted with dichloromethane. The organic layer was washed with saturated aqueous sodium chloride solution, dried over anhydrous magnesium sulfate, filtered and concentrated under reduced pressure. The concentrate was purified and concentrated by column chromatography to obtain 4-(5-(difluoromethyl)-1,3,4-oxadiazol-2-yl)pyridin-2(1*H*)-one (1.800 g, 49.6%) in the form of a white solid: **LRMS** (ES) *m*/*z* 213.96 [M+H]⁺, calculated MW 213.14.

### Step 3: Synthesis of 4-(5-(difluoromethyl)-1,3,4-oxadiazol-2-yl)-1-(prop-2-yn-1-yl)pyridin-2(1H)-one

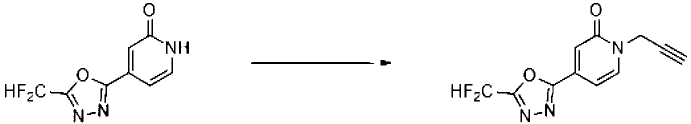

4-(5-(Difluoromethyl)-1,3,4-oxadiazol-2-yl)pyridin-2(1*H*)-one (2.500 g, 11.729 mmol), 3-bromoprop-1-yne (2.093 g, 17.594 mmol) and potassium carbonate (4.863 g, 35.188 mmol) were dissolved in N,N-dimethylformamide (50 mL) at room temperature, and the obtained solution was stirred at the same temperature for 18 hours. The reaction mixture was filtered through a Celite pad to remove solids, and water was added to the filtrate and extracted with ethyl acetate. The organic layer was washed with saturated aqueous sodium chloride solution, dried over anhydrous sodium sulfate, filtered and concentrated under reduced pressure. The concentrate was purified and concentrated by column chromatography to obtain 4-(5-(difluoromethyl)-1,3,4-oxadiazol-2-yl)-1-(prop-2-yn-1-yl)pyridin-2(1*H*)-one (2.000 g, 67.9%) in the form of a yellow solid: **LRMS** (ES) *m*/*z* 293.16 [M+ACN+H]⁺, calculated MW 251.19.

### Step 4: Synthesis of 4-f5-(difluoromethyl)-1,3,4-oxadiazol-2-yl)-1-(3-(pyridin-4-yl)prop-2-yn-1-yl)pyridin-2(1H)-one

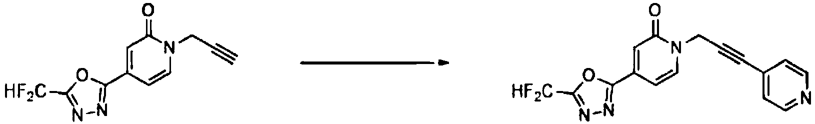

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 4-iodopyridine (0.033 g, 0.159 mmol) as starting materials were used in a similar manner to Step 4 of Example 2 to obtain 4-(5-(difluoromethyl)-1,3,4-oxadiazol-2-yl)-1-(3-(pyridin-4-yl)prop-2-yn-1-yl)pyridin-2(1*H*)-one (0.028 g, 53.6%) in the form of a yellow solid: **LRMS** (ES) *m*/*z* 329.03 [M+H]⁺, calculated MW 328.28; **¹H-NMR** (400 MHz, CD₃OD) d 8.54 (m, 2 H), 8.07 (d, *J* = 7.2 Hz, 1 H), 7.47 (m, 2 H), 7.23 (t, *J* = 51.6 Hz, 1 H), 7.23 (d, *J* = 2.0 Hz, 1 H), 7.03 (dd, *J* = 7.0, 1.8 Hz, 1 H).

### Example 9: Synthesis of 4-(5-(difluoromethyl)-1,3,4-oxadiazol-2-yl)-1-(3-(pyridin-3-yl)prop-2-yn-1-yl)pyridin-2(1H)-one

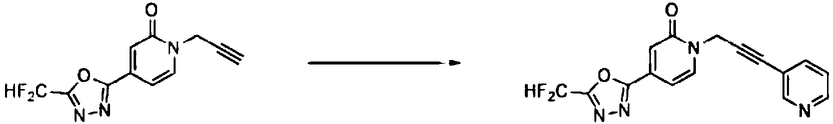

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 3-iodopyridine (0.033 g, 0.159 mmol) as starting materials were used in a similar manner to Step 4 of Example 2 to obtain 4-(5-(difluoromethyl)-1,3,4-oxadiazol-2-yl)-1-(3-(pyridin-3-yl)prop-2-yn-1-yl)pyridin-2(1H)-one (0.030 g, 57.4%) in the form of a yellow solid: **LRMS** (ES) *m*/*z* 329.10 [M+H]⁺, calculated MW 328.28; **¹H-NMR** (400 MHz, DMSO-*d*₆) d 8.64 ~ 8.52 (m, 2 H), 8.09 (d, *J* = 7.6 Hz, 1 H), 7.91 (d, *J* = 6.8 Hz, 1 H), 7.42 (m, 1 H), 7.23 (t, *J* = 51.8 Hz, 1 H), 7.23 (m, 1 H), 7.04 (dd, *J* = 7.4, 1.8 Hz, 1 H), 5.11 (s, 2 H).

### Example 10: Synthesis of 4-(5-(difluoromethyl)-1,3,4-oxadiazol-2-yl)-1-(3-(pyridin-2-yl)prop-2-yn-1-yl)pyridin-2(1H)-one

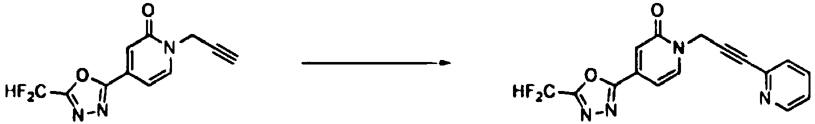

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 2-iodopyridine (0.033 g, 0.159 mmol) as starting materials were used in a similar manner to Step 4 of Example 2 to obtain 4-(5-(difluoromethyl)-1,3,4-oxadiazol-2-yl)-1-(3-(pyridin-2-yl)prop-2-yn-1-yl)pyridin-2(1H)-one (0.020 g, 38.3%) in the form of a yellow solid: **LRMS** (ES) *m*/*z* 329.03 [M+H]⁺, calculated MW 328.28; **¹H-NMR** (400 MHz, DMSO-*d*₆) d 8.51 (m, 1 H), 8.13 (d, *J* = 7.2 Hz, 1 H), 7.85 (t, *J* = 7.8 Hz, 1 H), 7.67 ~ 7.56 (m, 2 H), 7.42 (dd, *J* = 7.8, 5.0 Hz, 1 H), 7.24 (t, *J* = 51.8 Hz, 1 H), 7.24 (m, 1 H), 7.05 (d, *J* = 6.8 Hz, 1 H), 5.14 (s, 2 H).

### Example 11: Synthesis of 4-(5-(difluoromethyl)-1,3,4-oxadiazol-2-yl)-1-(3-(4-fluorophenyl)prop-2-yn-1-yl)pyridin-2(1H)-one

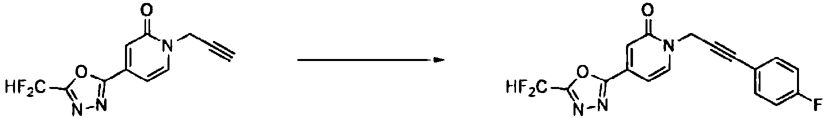

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-fluoro-4-iodobenzene (0.035 g, 0.159 mmol) as starting materials were used in a similar manner to Step 4 of Example 2 to obtain4-(5-(difluoromethyl)-1,3,4-oxadiazol-2-yl)-1-(3-(4-fluorophenyl)prop-2-yn-1-yl)pyridin-2(1H)-one (0.010 g, 18.2%) in the form of an ivory solid: LRMS (ES) *m*/*z* 346.04 [M+H]⁺, calculated MW 345.28; **¹H-NMR** (400 MHz, CD₃OD) d 8.12 (dd, *J* = 6.8, 3.6 Hz, 1 H), 7.52 ~ 7.49 (m, 2 H), 7.25 (t, *J* = 51.8 Hz, 1 H), 7.26 ~ 7.23 (m, 1 H), 7.13 ~ 7.04 (m, 3 H), 5.08 (d, *J* = 3.6 Hz, 2 H).

### Example 12: Synthesis of 4-(5-(difluoromethyl)-1,3,4-oxadiazol-2-yl)-1-(3-(3-fluorophenyl)prop-2-yn-1-yl)pyridin-2(1H)-one

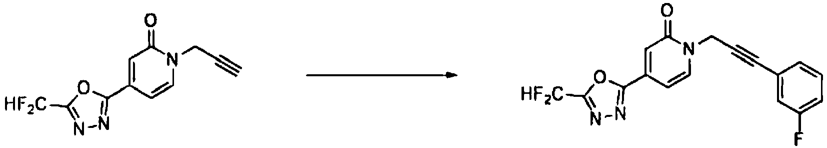

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-fluoro-3-iodobenzene (0.035 g, 0.159 mmol) as starting materials were used in a similar manner to Step 4 of Example 2 to obtain 4-(5-(difluoromethyl)-1,3,4-oxadiazol-2-yl)-1-(3-(3-fluorophenyl)prop-2-yn-1-yl)pyridin-2(1*H*)-one (0.012 g, 21.8%) in the form of an ivory solid: **LRMS** (ES) *m*/*z* 346.04 [M+H]⁺, calculated MW 345.28; **¹H-NMR** (400 MHz, CD₃OD) d 8.11 (d, *J* = 7.2 Hz, 1 H), 7.40 ~ 7.34 (m, 1 H), 7.28 (d, *J* = 19.6 Hz, 1 H), 7.25 (t, *J* = 51.8 Hz, 1H), 7.25 ~ 7.21 (m, 2 H), 7.16 ~ 7.11 (m, 1 H), 7.06 (dd, *J* = 7.4, 1.8 Hz, 1 H), 5.10 (s, 2 H).

### Example 13: Synthesis of 4-(5-(difluoromethyl)-1,3,4-oxadiazol-2-yl)-1-(3-(2-fluorophenyl)prop-2-yn-1-yl)pyridin-2(1H)-one

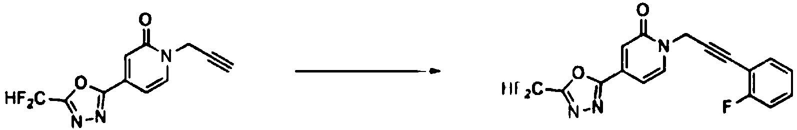

4-(5-(Difluoromethyl)-1,3,4-oxadiazol-2-yl)-1-(prop-2-yn-1-yl)pyridin-2(1H)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-fluoro-2-iodobenzene (0.035 g, 0.159 mmol) as starting materials were used in a similar manner to Step 4 of Example 2 to obtain 4-(5-(difluoromethyl)-1,3,4-oxadiazol-2-yl)-1-(3-(2-fluorophenyl)prop-2-yn-1-yl)pyridin-2(1*H*)-one (0.010 g, 18.2%) in the form of an ivory solid: **LRMS** (ES) *m*/*z* 346.04 [M+H]⁺, calculated MW 345.28; **¹H-NMR** (400 MHz, CD₃OD) d 8.12 (d, *J* = 6.8 Hz, 1 H), 7.50 ~ 7.49 (m, 1 H), 7.40 ~ 7.38 (m, 1 H), 7.25 (m, 1 H), 7.25 (t, *J* = 51.8 Hz, 1 H), 7.17 ~ 7.12 (m, 2 H), 7.08 ~ 7.06 (m, 1 H), 5.15 (s, 2 H).

### Example 14: Synthesis of 1-(3-(4-chlorophenyl)prop-2-yn-1-yl)-4-(5-(difluoromethyl)-1,3,4-oxadiazol-2-yl)pyridin-2(1H)-one

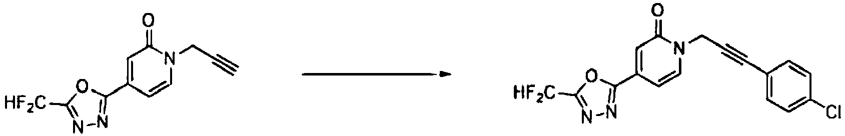

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-chloro-4-iodobenzene (0.038 g, 0.159 mmol) as starting materials were used in a similar manner to Step 4 of Example 2 to obtain 1-(3-(4-chlorophenyl)prop-2-yn-1-yl)-4-(5-(difluoromethyl)-1,3,4-oxadiazol-2-yl)pyridin-2(1H)-one (0.010 g, 17.4%) in the form of an ivory solid: **LRMS** (ES) *m*/*z* 362.00 [M+H]⁺, calculated MW 361.73; **¹H-NMR** (400 MHz, CD₃OD) d 8.10 (d, *J* = 6.8 Hz, 1 H), 7.46 ~ 7.44 (m, 2 H), 7.35 (m, 2 H), 7.25 (t, *J* = 51.8 Hz, 1 H), 7.23 (m, 1 H), 7.05 (dd, *J* = 6.8, 2.0 Hz, 1 H), 5.08 (s, 2 H).

### Example 15: Synthesis of 1-(3-(3-chlorophenyl)prop-2-yn-1-yl)-4-(5-(difluoromethyl)-1,3,4-oxadiazol-2-yl)pyridin-2(1H)-one

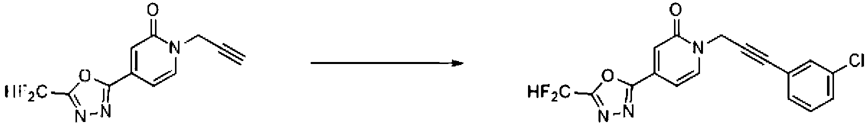

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-chloro-3-iodobenzene (0.038 g, 0.159 mmol) as starting materials were used in a similar manner to Step 4 of Example 2 to obtain 1-(3-(3-chlorophenyl)prop-2-yn-1-yl)-4-(5-(difluoromethyl)-1,3,4-oxadiazol-2-yl)pyridin-2(1*H*)-one (0.010 g, 17.4%) in the form of an ivory solid: **LRMS** (ES) *m*/*z* 362.00 [M+H]⁺, calculated MW 361.73; **¹H-NMR** (400 MHz, CD₃OD) d 8.11 (d, *J* = 7.6 Hz, 1 H), 7.50 (t, *J* = 1.6 Hz, 1 H), 7.41 ~ 7.36 (m, 2 H), 7.25 (t, *J* = 51.8 Hz, 1 H), 7.24 (d, *J =* 2.0 Hz, 2 H), 7.06 (dd, *J* = 7.0, 2.2 Hz, 1 H), 5.10 (s, 2 H).

### Example 16: Synthesis of 1-(3-(2-chlorophenyl)prop-2-yn-1-yl)-4-(5-(difluoromethyl)-1,3,4-oxadiazol-2-yl)pyridin-2(1H)-one

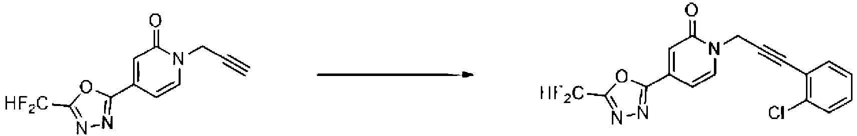

4-(5-(Difluoromethyl)-1,3,4-oxadiazol-2-yl)-1-(prop-2-yn-1-yl)pyridin-2(1H)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-chloro-2-iodobenzene (0.038 g, 0.159 mmol) as starting materials were used in a similar manner to Step 4 of Example 2 to obtain 1-(3-(2-chlorophenyl)prop-2-yn-1-yl)-4-(5-(difluoromethyl)-1,3,4-oxadiazol-2-yl)pyridin-2(1H)-one (0.008 g, 13.9%) in the form of an ivory solid: **LRMS** (ES) *m*/*z* 362.00 [M+H]⁺, calculated MW 361.73; **¹H-NMR** (400 MHz, CD₃OD) d 8.17 (m, 1 H), 7.56 ~ 7.08 (m, 7 H), 5.16 (s, 2 H).

### Example 17: Synthesis of 4-(5-(difluoromethyl)-1,3,4-oxadiazol-2-yl)-1-(3-(p-tolyl)prop-2-yn-1-yl)pyridin-2(1H)-one

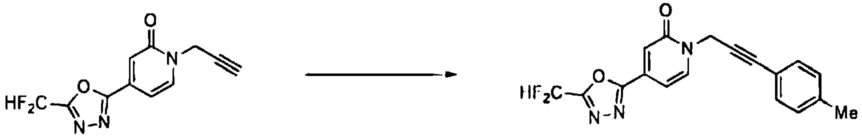

4-(5-(Difluoromethyl)-1,3,4-oxadiazol-2-yl)-1-(prop-2-yn-l-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-iodo-4-methylbenzene (0.035 g, 0.159 mmol) as starting materials were used in a similar manner to Step 4 of Example 2 to obtain 4-(5-(difluoromethyl)-1,3,4-oxadiazol-2-yl)-1-(3-(p-tolyl)prop-2-yn-1-yl)pyridin-2(1*H*)-one (0.010 g, 18.4%) in the form of an ivory solid: **LRMS** (ES) *m*/*z* 342.19 [M+H]⁺, calculated MW 341.32; **¹H-NMR** (400 MHz, CD₃OD) d 8.12 (d, *J* = 7.2 Hz, 1 H), 7.25 (t, *J* = 51.8 Hz, 1 H), 7.34 (d, *J* = 7.6 Hz, 2 H), 7.25 ~ 7.23 (m, 1 H), 7.16 (d, *J* = 7.6 Hz, 2 H), 7.05 (dd, *J* = 7.0, 2.2 Hz, 1 H), 5.07 (s, 2 H), 2.34 (s, 3 H).

### Example 18: Synthesis of 4-(5-(difluoromethyl)-1,3,4-oxadiazol-2-yl)-1-(3-(m-tolyl)prop-2-yn-1-yl)pyridin-2(1H)-one

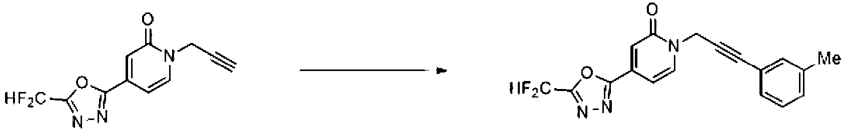

4-(5-(Difluoromethyl)-1,3,4-oxadiazol-2-yl)-1-(prop-2-yn-1-yl)pyridin-2(1*H*)-one (0.040 g, 0.159 mmol), triethylamine (0.111 mL, 0.796 mmol), bis(triphenylphosphine)palladium(II) dichloride (0.006 g, 0.008 mmol), copper iodide (CuI, 0.004 g, 0.019 mmol) and 1-iodo-3-methylbenzene (0.035 g, 0.159 mmol) as starting materials were used in a similar manner to Step 4 of Example 2 to obtain 4-(5-(difluoromethyl)-1,3,4-oxadiazol-2-yl)-1-(3-(m-tolyl)prop-2-yn-1-yl)pyridin-2(1*H*)-one (0.015 g, 27.6%) in the form of an ivory solid: L**RMS** (ES) *m*/*z* 342.05 [M+H]⁺, calculated MW 341.32; **¹H-NMR** (400 MHz, CD₃OD) d 8.13 (d, *J =* 7.2 Hz, 1 H), 7.25 (t, *J* = 51.6 Hz, 1 H), 7.30 ~ 7.21 (m, 5 H), 7.07 (d, *J* = 7.2 Hz, 1 H), 5.08 (s, 2 H), 2.32 (s, 3 H).

### Example 19: Synthesis of 4-(5-(difluoromethyl)-1,3,4-oxadiazol-2-yl)-1-(3-(3-(trifluoromethyl)phenyl)prop-2-yn-1-yl)pyridin-2(1H)-one

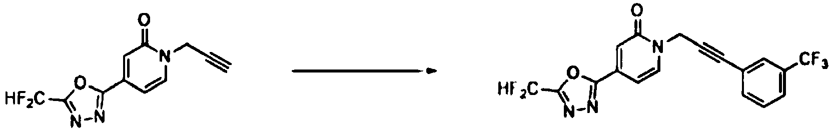

4-(5-(Difluoromethyl)-1,3,4-oxadiazol-2-yl)-1-(prop-2-yn-1-yl)pyridin-2(1H)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 1-iodo-3-(trifluoromethyl)benzene (0.032 mL, 0.219 mmol) as starting materials were used in a similar manner to Step 4 of Example 2 to obtain 4-(5-(difluoromethyl)-1,3,4-oxadiazol-2-yl)-1-(3-(3-(trifluoromethyl)phenyl)prop-2-yn-1-yl)pyridin-2(1H)-one (0.030 g, 38.1%) in the form of an ivory solid: **LRMS** (ES) *m*/*z* 396.27 [M+H]⁺, calculated MW 395.29; **¹H-NMR** (400 MHz, DMSO-*d*₆) d 8.12 (d, *J* = 6.8 Hz, 1 H), 7.79 (s, 1 H), 7.75 (d, *J* = 7.6 Hz, 2 H), 7.53 (t, *J* = 51.2 Hz, 1 H), 7.60 (t, *J* = 7.8 Hz, 1 H), 7.01 (d, *J* = 1.6 Hz, 1 H), 6.84 (dd, *J* = 7.4, 1.8 Hz, 1 H), 5.06 (s, 2 H).

### Example 20: Synthesis of 4-(5-(difluoromethyl)-1,3,4-oxadiazol-2-yl)-1-(3-(3-(trifluoromethoxy)phenyl)prop-2-yn-1-yl)pyridin-2(1H)-one

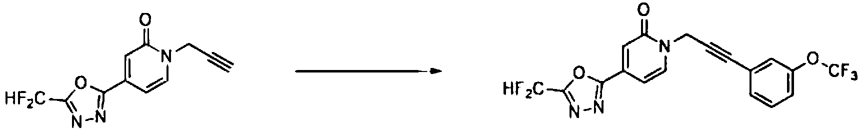

4-(5-(Difluoromethyl)-1,3,4-oxadiazol-2-yl)-1-(prop-2-yn-l-yl)pyridin-2(1H)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 1-iodo-3-(trifluoromethoxy)benzene (0.034 mL, 0.219 mmol) as starting materials were used in a similar manner to Step 4 of Example 2 to obtain 4-(5-(difluoromethyl)-1,3,4-oxadiazol-2-yl)-1-(3-(3-(trifluoromethoxy)phenyl)prop-2-yn-1-yl)pyridin-2(1*H*)-one (0.030 g, 36.6%) in the form of an ivory solid: **LRMS** (ES) *m*/*z* 411.93 [M+H]⁺, calculated MW 411.29; **¹H-NMR** 400 MHz, DMSO-*d*₆) d 8.10 (d, *J* = 7.2 Hz, 1 H), 7.53 (t, *J* = 51.4 Hz, 1 H), 7.50 ~ 7.40 (m, 4 H), 7.01 (d, *J* = 2.4 Hz, 1 H), 6.84 (dd, *J* = 7.2, 2.0 Hz, 1 H), 5.05 (s, 2 H).

### Example 21: Synthesis of 4-(5-(difluoromethyl)-1,3,4-oxadiazol-2-yl)-1-(3-(3-methoxyphenyl)prop-2-yn-1-yl)pyridin-2(1H)-one

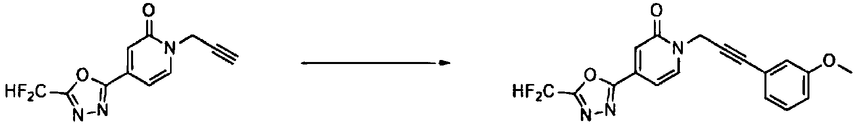

4-(5-(Difluoromethyl)-1,3,4-oxadiazol-2-yl)-1-(prop-2-yn-1-yl)pyridin-2(1*H*)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 1-iodo-3-methoxybenzene (0.029 mL, 0.219 mmol) as starting materials were used in a similar manner to Step 4 of Example 2 to obtain 4-(5-(difluoromethyl)-1,3,4-oxadiazol-2-yl)-1-(3-(3-methoxyphenyl)prop-2-yn-1-yl)pyridin-2(1H)-one (0.013 g, 18.3%) in the form of an ivory solid: **LRMS** (ES) *m*/*z* 358.16 [M+H]⁺, calculated MW 357.32; **¹H-NMR** (400 MHz, DMSO-*d*₆) d 8.10 (d, *J* = 7.2 Hz, 1 H), 7.53 (t, *J* = 51.6 Hz, 1 H), 7.26 (t, *J* = 7.8 Hz, 1 H), 7.01 ~ 6.93 (m, 4 H), 6.84 (dd, *J=* 7.0, 2.2 Hz, 1 H), 5.02 (s, 2 H), 3.72 (s, 3 H).

### Example 22: Synthesis of 1-(3-(6-chloropyridin-2-yl)prop-2-yn-1-yl)-4-(5-(difluoromethyl)-1,3,4-oxadiazol-2-yl)pyridin-2(1H)-one

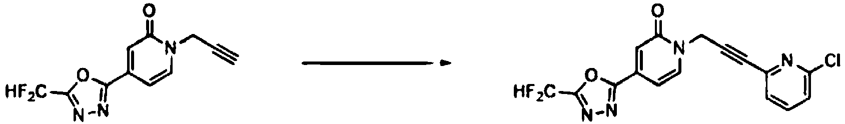

4-(5-(Difluoromethyl)-1,3,4-oxadiazol-2-yl)-1-(prop-2-yn-1-yl)pyridin-2(1H)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 2-chloro-6-iodopyridine (0.026 mL, 0.219 mmol) as starting materials were used in a similar manner to Step 4 of Example 2 to obtain 1-(3-(6-chloropyridin-2-yl)prop-2-yn-1-yl)-4-(5-(difluoromethyl)-1,3,4-oxadiazol-2-yl)pyridin-2(1*H*)-one (0.028 g, 38.8%) in the form of a yellow solid: **LRMS** (ES) *m*/*z* 363.12 [M+H]⁺, calculated MW 362.72; **¹H-NMR** (400 MHz, DMSO-*d*₆) d 8.09 (d, *J* = 6.4 Hz, 1 H), 7.85 (t, *J* = 7.8 Hz, 1 H), 7.53 (t, *J* = 51.2 Hz, 1 H), 7.57 ~ 7.50 (m, 2 H), 7.02 (d, *J* = 1.6 Hz, 1 H), 6.85 (dd, *J* = 7.0, 2.2 Hz, 1 H), 5.08 (s, 2 H).

### Example 23: Synthesis of 1-(3-(2-chloropyridin-4-yl)prop-2-yn-1-yl)-4-(5-(difluoromethyl)-1,3,4-oxadiazol-2-yl)pyridin-2(1H)-one

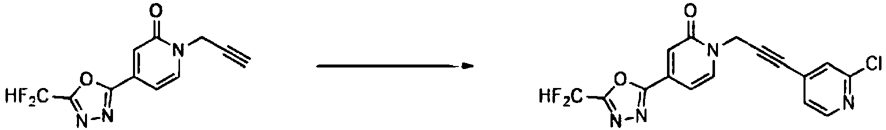

4-(5-(Difluoromethyl)-1,3,4-oxadiazol-2-yl)-1-(prop-2-yn-l-yl)pyridin-2(1H)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 2-chloro-4-iodopyridine (0.026 mL, 0.219 mmol) as starting materials were used in a similar manner to Step 4 of Example 2 to obtain 1-(3-(2-chloropyridin-4-yl)prop-2-yn-1-yl)-4-(5-(difluoromethyl)-1,3,4-oxadiazol-2-yl)pyridin-2(1*H*)-one (0.015 g, 20.8%) in the form of a yellow solid: **LRMS** (ES) *m*/*z* 363.12 [M+H]⁺, calculated MW 362.72; **¹H-NMR** (400 MHz, DMSO-*d*₆) d 8.40 (d, *J* = 6.0 Hz, 1 H), 8.09 (d, *J* = 6.4 Hz, 1 H), 7.53 (t, *J* = 51.2 Hz, 1 H), 7.61 (m, 1 H), 7.44 (dd, *J* = 5.0, 1.4 Hz, 1 H), 7.02 (d, *J* = 1.2 Hz, 1 H), 6.84 (dd, *J* = 7.4, 1.8 Hz, 1 H), 5.10 (s, 2 H).

### Example 24: Synthesis of 4-(5-(difluoromethyl)-1,3,4-oxadiazol-2-yl)-1-(3-(6-methylpyridin-2-yl)prop-2-yn-1-yl)pyridin-2(1H)-one

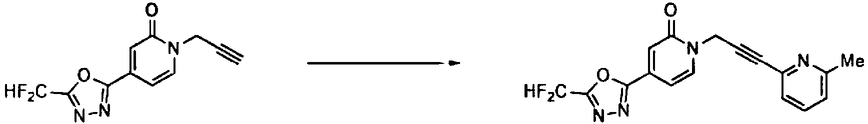

4-(5-(Difluoromethyl)-1,3,4-oxadiazol-2-yl)-1-(prop-2-yn-l-yl)pyridin-2(1*H*)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 2-iodo-6-methylpyridine (0.026 mL, 0.219 mmol) as starting materials were used in a similar manner to Step 4 of Example 2 to obtain 4-(5-(difluoromethyl)-1,3,4-oxadiazol-2-yl)-1-(3-(6-methylpyridin-2-yl)prop-2-yn-1-yl)pyridin-2(1*H*)-one (0.020 g, 29.4%) in the form of a brown solid: **LRMS** (ES) *m*/*z* 343.17 [M+H]⁺, calculated MW 342.31; **¹H-NMR** (400 MHz, DMSO-*d*₆) d 8.09 (d, *J* = 7.2 Hz, 1 H), 7.68 ~ 7.64 (m, 1 H), 7.53 (t, *J* = 51.8 Hz, 1 H), 7.31 (d, *J* = 7.2 Hz, 1 H), 7.23 (d, *J* = 8.0 Hz, 1 H), 7.02 (d, *J* = 1.2 Hz, 1 H), 6.85 (dd, *J* = 7.0, 2.2 Hz, 1 H), 5.05 (s, 2 H), 2.40 (s, 3 H).

### Example 25: Synthesis of 1-(3-(3-bromophenyl)prop-2-yn-1-yl)-4-(5-(difluoromethyl)-1,3,4-oxadiazol-2-yl)pyridin-2(1H)-one

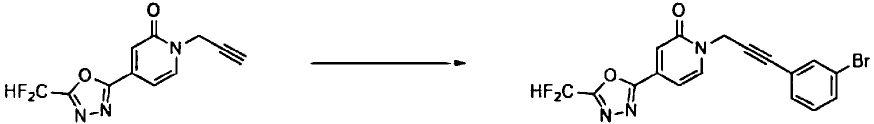

4-(5-(Difluoromethyl)-1,3,4-oxadiazol-2-yl)-1-(prop-2-yn-1-yl)pyridin-2(1*H*)-one (0.050 g, 0.199 mmol), triethylamine (0.139 mL, 0.995 mmol), bis(triphenylphosphine)palladium(II) dichloride (0.007 g, 0.010 mmol), copper iodide (CuI, 0.005 g, 0.024 mmol) and 1-bromo-3-iodobenzene (0.028 mL, 0.219 mmol) as starting materials were used in a similar manner to Step 4 of Example 2 to obtain 1-(3-(3-bromophenyl)prop-2-yn-1-yl)-4-(5-(difluoromethyl)-1,3,4-oxadiazol-2-yl)pyridin-2(1*H*)-one (0.019 g, 23.5%) in the form of a white solid: **LRMS** (ES [M+2H]) *m*/*z* 408.10 [M+H]⁺, calculated MW 406.19; **¹H-NMR** (400 MHz, DMSO-*d*₆) d 8.10 (d, *J* = 6.8 Hz, 1 H), 7.65 (d, *J* = 1.6 Hz, 1 H), 7.59 (dt, *J* = 6.8, 1.3 Hz, 1 H), 7.53 (t, *J* = 51.4 Hz, 1 H), 7.45 (dd, *J* = 7.6, 1.2 Hz, 1 H), 7.31 (t, *J* = 8.0 Hz, 1 H), 7.01 (d, *J* = 1.2 Hz, 1 H), 6.84 (dd, *J* = 7.2, 2.0 Hz, 1 H), 5.04 (s, 2 H).

### Example 26: Synthesis of 4-(5-(difluoromethyl)-1,3,4-oxadiazol-2-yl)-1-(3-(3-(difluoromethyl)phenyl)prop-2-yn-1-yl)pyridin-2(1H)-one

### Step 1: Synthesis of 3-(3-(difluoromethyl)phenyl)prop-2-yn-1-ol

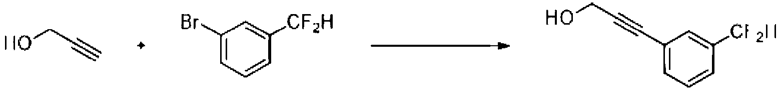

3-(3-(Difluoromethoxy)phenyl)prop-2-yn-l-ol (0.550 g, 2.775 mmol), methanesulfonyl chloride (0.322 mL, 4.163 mmol) and triethylamine (0.774 mL, 5.551 mmol) were dissolved in dichloromethane (10 mL) at room temperature and stirred at the same temperature for 1 hour. Water was poured into the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 3-(3-(difluoromethoxy)phenyl)prop-2-yn-1-yl methanesulfonate (0.430 g, 56.1%) in the form of a yellow liquid: **LRMS** (ES) *m*/*z* no detection [M+H]⁺, calculated MW 182.17.

### Step 2: Synthesis of 3-(3-(difluoromethyl)phenyl)prop-2-yn-1-yl methanesulfonate

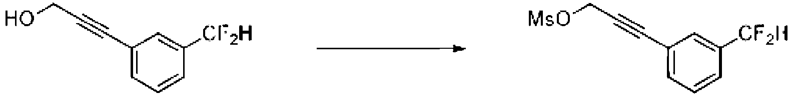

3-(3-(Difluoromethyl)phenyl)prop-2-yn-1-ol (0.750 g, 4.117 mmol), methanesulfonyl chloride (0.478 mL, 6.176 mmol) and triethylamine (1.148 mL, 8.234 mmol) were dissolved in dichloromethane (10 mL) at room temperature and stirred at the same temperature for 1 hour. Water was poured into the reaction mixture and extracted with dichloromethane. The organic layer was washed with saturated aqueous sodium chloride solution, dried over anhydrous magnesium sulfate, filtered and concentrated under reduced pressure. The concentrate was purified and concentrated by column chromatography to obtain 3-(3-(difluoromethyl)phenyl)prop-2-yn-1-yl methanesulfonate (0.975 g, 91.0%) in the form of a transparent liquid: **LRMS** (ES) *m*/*z* no detection [M+H]⁺, calculated MW 260.25.

### Step 3: Synthesis of 4-(5-(difluoromethyl)-1,3,4-oxadiazol-2-yl)-1-(3-(3-(difluoromethyl)phenyl)prop-2-yn-1-yl)pyridin-2(1H)-one

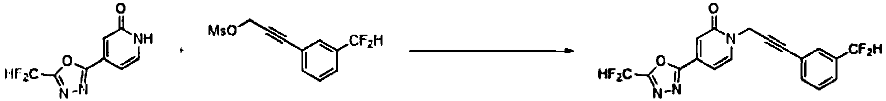

4-(5-(Difluoromethyl)-1,3,4-oxadiazol-2-yl)pyridin-2(1*H*)-one (0.050 g, 0.235 mmol), 3-(3-(difluoromethyl)phenyl)prop-2-yn-1-yl methanesulfonate (0.092 g, 0.352 mmol) and cesium carbonate (Cs₂CO₃, 0.115 g, 0.352 mmol) were dissolved in acetonitrile (2 mL) at room temperature and stirred at the same temperature for 10 minutes. Water was poured into the reaction mixture and extracted with dichloromethane. The organic layer was washed with saturated aqueous sodium chloride solution, dried over anhydrous magnesium sulfate, filtered and concentrated under reduced pressure. The concentrate was purified and concentrated by column chromatography to obtain 4-(5-(difluoromethyl)-1,3,4-oxadiazol-2-yl)-1-(3-(3-(difluoromethyl)phenyl)prop-2-yn-1-yl)pyridin-2(1H)-one (0.037 g, 41.5%) in the form of a white solid: **LRMS** (ES) *m*/*z* 378.17 [M+H]⁺, calculated MW 377.3; **¹H-NMR** 400 MHz, CD₃OD) d 8.09 (d, *J* = 7.2 Hz, 1 H), 7.61 (s, 1 H), 7.58 (d, *J =* 7.2 Hz, 1 H), 7.53 (d, *J* = 7.6 Hz, 1 H), 7.46 (t, *J* = 7.6 Hz, 1 H), 7.23 (t, *J* = 51.8 Hz, 1 H), 7.22 (s, 1 H), 7.03 (dd, *J* = 7.0, 1.4 Hz, 1 H), 6.73 (t, *J* = 56.2 Hz, 1 H), 5.08 (s, 2 H).

### Example 27: Synthesis of 1-(3-(3-(difluoromethoxy)phenyl)prop-2-yn-1-yl)-4-(5-(difluoromethyl)-1,3,4-oxadiazol-2-yl)pyridin-2(1H)-one

### Step 1: Synthesis of 3-(3-(difluoromethoxy)phenyl)prop-2-yn-1-ol

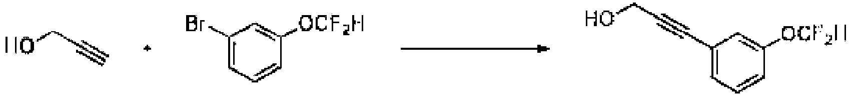

1-Bromo-3-(difluoromethoxy)benzene (1.000 g, 4.484 mmol), 2,3,4,6,7,8,9,10-octahydropyrimido[1,2-a]azepine (DBU, 0.805 mL, 5.381 mmol), tetrakis(triphenylphosphine)palladium (0.155 g, 0.135 mmol) and copper iodide (CuI, 0.026 g, 0.135 mmol) as starting materials were used in a similar manner to Step 1 of Example 26 to obtain 3-(3-(difluoromethoxy)phenyl)prop-2-yn-1-ol (0.550 g, 61.9%) in the form of a yellow solid: **LRMS** (ES) *m*/*z* no detection [M+H]⁺, calculated MW 198.17.

### Step 2: Synthesis of 3-(3-(difluoromethoxy)phenyl)prop-2-yn-1-yl methanesulfonate

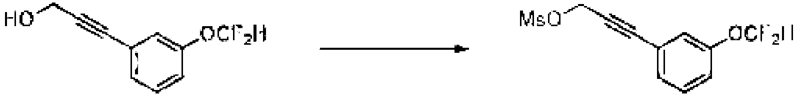

3-(3-(Difluoromethoxy)phenyl)prop-2-yn-1-ol (0.550 g, 2.775 mmol), methanesulfonyl chloride (0.322 mL, 4.163 mmol) and triethylamine (0.774 mL, 5.551 mmol) as starting materials were used in a similar manner to Step 2 of Example 26 to obtain 3-(3-(difluoromethoxy)phenyl)prop-2-yn-1-yl methanesulfonate (0.430 g, 56.1%) in the form of a yellow liquid: **LRMS** (ES) *m*/*z* no detection [M+H]⁺, calculated MW 276.25.

### Step 3: Synthesis of 1-(3-(3-(difluoromethoxy)phenyl)prop-2-yn-1-yl)-4-(5-(difluoromethyl)-1,3,4-oxadiazol-2-yl)pyridin-2(1H)-one

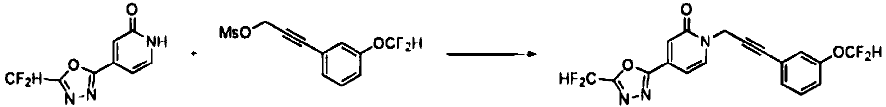

4-(5-(Difluoromethyl)-1,3,4-oxadiazol-2-yl)pyridin-2(1*H*)-one (0.050 g, 0.235 mmol), 3-(3-(difluoromethoxy)phenyl)prop-2-yn-1-yl methanesulfonate (0.097 g, 0.352 mmol) and cesium carbonate (Cs₂CO₃, 0.153 g, 0.469 mmol) as starting materials were used in a similar manner to Step 3 of Example 26 to obtain 1-(3-(3-(difluoromethoxy)phenyl)prop-2-yn-1-yl)-4-(5-(difluoromethyl)-1,3,4-oxadiazol-2-yl)pyridin-2(1*H*)-one (0.007 g, 7.6%) in the form of a brown solid: **LRMS** (ES) *m*/*z* 394.29 [M+H]⁺, calculated MW 393.3; **¹H-NMR** (400 MHz, CD₃OD) d 8.08 (d, *J* = 7.2 Hz, 1 H), 7.22 (t, *J* = 51.6 Hz, 1 H), 7.38 ~ 7.29 (m, 2 H), 7.22 (d, *J* = 1.6 Hz, 2 H), 7.14 (d, *J* = 8.0 Hz, 1 H), 7.03 (dd, *J* = 7.2, 2.0 Hz, 1 H), 6.82 (t, J*=* 73.8 Hz, 1 H), 5.07 (s, 2 H).

### Example 28: Synthesis of 1-(3-(3-chlorophenyl)prop-2-yn-1-yl)-4-(5-(trifluoromethyl)-1,3,4-oxadiazol-2-yl)pyridin-2(1H)-one

### Step 1: Synthesis of 4-(5-(trifluoromethyl)-1,3,4-oxadiazol-2-yl)pyridin-2(1H)-one

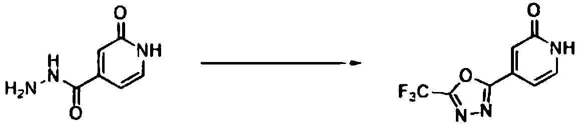

2-Oxo-1,2-dihydropyridine-4-carbohydrazide (1.730 g, 11.297 mmol), triethylamine (4.724 mL, 33.891 mmol) and trifluoroacetic anhydride (4.787 mL, 33.891 mmol) as starting materials were used in a similar manner to Step 2 of Example 8 to obtain 4-(5-(trifluoromethyl)-1,3,4-oxadiazol-2-yl)pyridin-2(1*H*)-one (1.500 g, 57.4%) in the form of a light brown solid: **¹H-NMR** (400 MHz, DMSO-*d*₆) d 8.91 (s, 1 H), 7.59 (s, 2 H).

### Step 2: Synthesis of 3-(3-chlorophenyl)prop-2-yn-1-ol

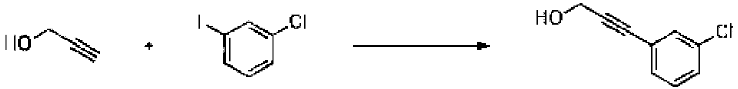

1-Chloro-3-iodobenzene (1.000 g, 4.194 mmol), 2,3,4,6,7,8,9,10-octahydropyrimido[1,2-a]azepine (DBU, 0.753) mL, 5.033 mmol), tetrakis(triphenylphosphine)palladium (0.145 g, 0.126 mmol) and copper iodide (CuI, 0.024 g, 0.126 mmol) as starting materials were used in a similar manner to Step 1 of Example 26 to obtain 3-(3-chlorophenyl)prop-2-yn-1-ol (0.580 g, 83.0%) in the form of a yellow solid: **LRMS** (ES) *m*/*z* no detection [M+H]⁺, calculated MW 166.60.

### Step 3: Synthesis of 3-(3-chlorophenyl)prop-2-yn-1-yl methanesulfonate

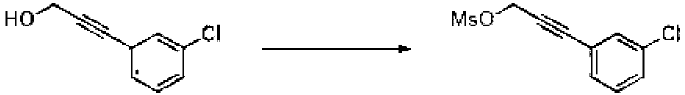

3-(3-Chlorophenyl)prop-2-yn-1-ol (0.580 g, 3.481 mmol), methanesulfonyl chloride (9.20 M solution in toluene, 0.568 mL, 5.222 mmol) and triethylamine (1.456 mL, 10.444 mmol) as starting materials were used in a similar manner to Step 2 of Example 26 to obtain 3-(3-chlorophenyl)prop-2-yn-1-yl methanesulfonate (0.650 g, 76.3%) in the form of a transparent liquid: **LRMS** (ES) *m*/*z* no detection [M+H]⁺, calculated MW 244.69.

### Step 4: Synthesis of 1-(3-(3-chlorophenyl)prop-2-yn-1-yl)-4-(5-(trifluoromethyl)-1,3,4-oxadiazol-2-yl)pyridin-2(1R)-one

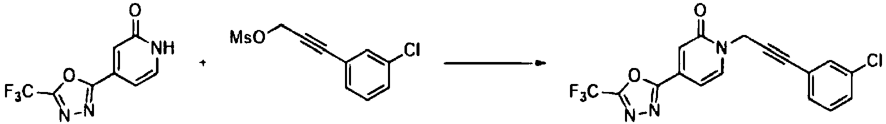

4-(5-(Trifluoromethyl)-1,3,4-oxadiazol-2-yl)pyridin-2(1*H*)-one (0.050 g, 0.216 mmol), 3-(3-chlorophenyl)prop-2-yn-1-yl methanesulfonate (0.079 g, 0.324 mmol) and cesium carbonate (Cs₂CO₃, 0.141 g, 0.433 mmol) as starting materials were used in a similar manner to Step 3 of Example 26 to obtain 1-(3-(3-chlorophenyl)prop-2-yn-1-yl)-4-(5-(trifluoromethyl)-1,3,4-oxadiazol-2-yl)pyridin-2(1*H*)-one (0.066 g, 80.3%) in the form of a yellow solid: **LRMS** (ES) *m*/*z* 380.09 [M+H]⁺, calculated MW 379.72; **¹H-NMR** (400 MHz, CD₃OD) d 8.09 (d, *J* = 6.8 Hz, 1 H), 7.46 (s, 1 H), 7.38 ~ 7.29 (m, 3 H), 7.23 (d, *J* = 1.6 Hz, 1 H), 7.03 (dd, *J* = 6.8, 1.6 Hz, 1 H), 5.07 (s, 2 H).

### Example 29: Synthesis of 4-(5-(difluoromethyl)-1,3,4-oxadiazol-2-yl)-1-(3-(5-methylpyridin-3-yl)prop-2-yn-1-yl)pyridin-2(1H)-one

### Step 1: Synthesis of 3-(5-methylpyridin-3-yl)prop-2-yn-1-ol

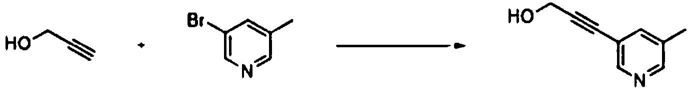

3-Bromo-5-methylpyridine (1.000 g, 5.813 mmol), 2,3,4,6,7,8,9,10-octahydropyrimido[1,2-a]azepine (DBU, 1.043 mL, 6.976 mmol), tetrakis(triphenylphosphine)palladium (0.202 g, 0.174 mmol), copper iodide (CuI, 0.033 g, 0.174 mmol) and prop-2-yn-1-ol (0.391 g, 6.976 mmol) as starting materials were used in a similar manner to Step 1 of Example 26 to obtain 3-(5-methylpyridin-3-yl)prop-2-yn-1-ol (0.752 g, 87.9%) in the form of a yellow solid: **LRMS** (ES) *m*/*z* no detection [M+H]⁺, calculated MW 147.18.

### Step 2: Synthesis of 4-(5-(difluoromethyl)-1,3,4-oxadiazol-2-yl)-1-(3-(5-methylpyridin-3-yl)prop-2-yn-1-yl)pyridin-2(1H)-one

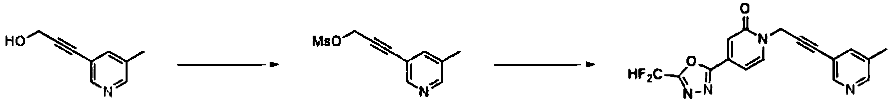

3-(5-Methylpyridin-3-yl)prop-2-yn-1-ol (0.050 g, 0.340 mmol), methanesulfonyl chloride (0.032 mL, 0.408 mmol) and triethylamine (0.057 mL, 0.408 mmol) were dissolved in acetonitrile (10 mL) and stirred at room temperature for 1 hour, and 4-(5-(difluoromethyl)-1,3,4-oxadiazol-2-yl)pyridin-2(1*H*)-one (0.109 g, 0.510 mmol) and cesium carbonate (Cs₂CO₃, 0.166 g, 0.510 mmol) were added thereto and stirred at the same temperature for additional 0.5 hours. Water was poured into the reaction mixture and extracted with dichloromethane. The organic layer was washed with saturated aqueous sodium chloride solution, dried over anhydrous sodium sulfate, filtered and concentrated under reduced pressure. The concentrate was purified and concentrated by column chromatography to obtain 4-(5-(difluoromethyl)-1,3,4-oxadiazol-2-yl)-1-(3-(5-methylpyridin-3-yl)prop-2-yn-1-yl)pyridin-2(1H)-one (0.020 g, 17.2%) in the form of a white solid: **LRMS** (ES) *m*/*z* 343.23 [M+H]⁺, calculated MW 342.31; **¹H-NMR** (400 MHz, CD₃OD) d 8.41 (s, 1 H), 8.35 (s, 1 H), 8.08 (d, *J* = 6.8 Hz, 1 H), 7.75 (s, 1 H), 7.62 ~ 7.56 (m, 1 H), 7.23 (t, *J* = 51.6 Hz, 1 H), 7.22 (d, *J* = 2.0 Hz, 1 H), 7.03 (dd, *J* = 7.2, 2.0 Hz, 1 H), 5.10 (s, 2 H), 2.32 (s, 3 H).

### Example 30: Synthesis of 1-(3-(3-chlorophenyl)prop-2-yn-1-yl)-4-(5-(difluoromethyl)-1,2,4-oxadiazol-3-yl)pyridin-2(1H)-one

### Step 1: Synthesis of N-hydroxy-2-oxo-1,2-dihydropyridine-4-carboximidamide

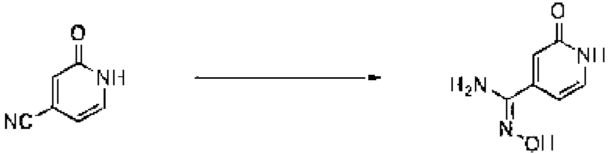

2-Oxo-1,2-dihydropyridine-4-carbonitrile (2.000 g, 16.651 mmol) and hydroxylamine (50.00% solution in water, 3.300 mL, 49.954 mmol) as starting materials were used in a similar manner to Step 1 of Example 8 to obtain N-hydroxy-2-oxo-1,2-dihydropyridine-4-carboximidamide (2.430 g, 95.3%) in the form of an ivory solid.

### Step 2: Synthesis of 4-(5-(difluoromethyl)-1,2,4-oxadiazol-3-yl)pyridin-2(1H)-one

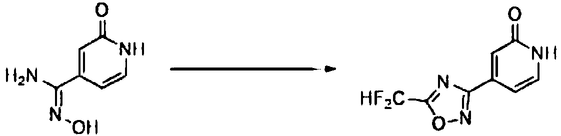

*N*'-hydroxy-2-oxo-1,2-dihydropyridine-4-carboximidamide (2.430 g, 15.868 mmol), triethylamine (6.635 mL, 47.604 mmol) and 2,2-difluoroacetic anhydride (5.918 mL, 47.604 mmol) as starting materials were used in a similar manner to Step 2 of Example 8 to obtain 4-(5-(difluoromethyl)-1,2,4-oxadiazol-3-yl)pyridin-2(1*H*)-one (2.000 g, 59.1%) in the form of a white solid.

### Step 3: Synthesis of 1-(3-(3-chlorophenyl)prop-2-yn-1-yl)-4-(5-(difluoromethyl)-1,2,4-oxadiazol-3-yl)pyridin-2(1H)-one

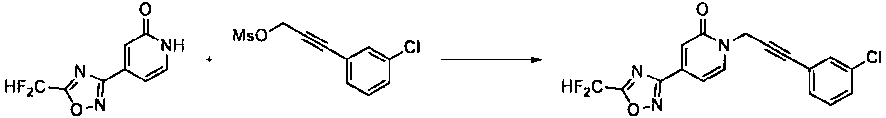

4-(5-(Difluoromethyl)-1,2,4-oxadiazol-3-yl)pyridin-2(1H)-one (0.050 g, 0.235 mmol), 3-(3-chlorophenyl)prop-2-yn-1-yl methanesulfonate (0.086 g, 0.352 mmol) and cesium carbonate (Cs₂CO₃, 0.153 g, 0.469 mmol) as starting materials were used in a similar manner to Step 3 of Example 26 to obtain 1-(3-(3-chlorophenyl)prop-2-yn-1-yl)-4-(5-(difluoromethyl)-1,2,4-oxadiazol-3-yl)pyridin-2(1H)-one (0.052 g, 61.3%) in the form of a white solid: **LRMS** (ES) *m*/*z* 362.19 [M+H]⁺, calculated MW 361.73; ¹H-NMR (400 MHz, CD₃OD) d 8.02 (d, *J =* 7.2 Hz, 1 H), 7.43 (s, 1 H), 7.36 ~ 7.25 (m, 3 H), 7.25 (d, *J =* 1.2 Hz, 1 H), 7.22 (t, *J =* 51.4 Hz, 1 H), 6.99 (dd, *J* = 7.2, 1.6 Hz, 1 H), 5.06 (s, 2 H).

### Example 31: Synthesis of 3-(3-(4-(5-(difluoromethyl)-1,3,4-oxadiazol-2-yl)-2-oxopyridin-1(2H)-yl)prop-1-yn-1-yl)benzonitrile

### Step 1: Synthesis of 3-(3-hydroxyprop-1-yn-1-yl)benzonitrile

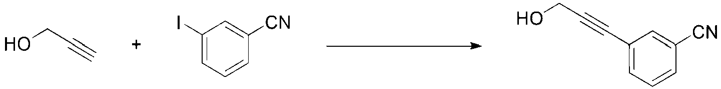

Prop-2-yn-1-ol (0.312 mL, 5.351 mmol), 3-iodobenzonitrile (1.226 g, 5.351 mmol), bis(triphenylphosphine)palladium(II) dichloride (0.075 g, 0.107 mmol), copper iodide (CuI, 0.020 g, 0.107 mmol) and triethylamine (2.238 mL, 16.054 mmol) as starting materials were used in a similar manner to Step 1 of Example 26 to obtain 3-(3-hydroxyprop-1-yn-1-yl)benzonitrile (0.768 g, 91.3%) in the form of a yellow oil: LRMS (ES) *m*/*z* no detection [M+H]⁺, calculated MW 235.26.

### Step 2: Synthesis of 3-(3-cyanophenyl)prop-2-yn-1-yl methanesulfonate

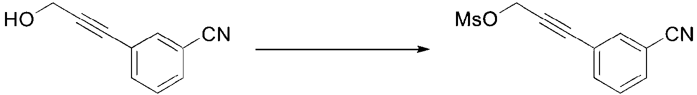

3-(3-Hydroxyprop-1-yn-1-yl)benzonitrile (0.768 g, 4.886 mmol), triethylamine (2.043 mL, 14.659 mmol) and methanesulfonyl chloride (0.756 mL, 9.773 mmol) as starting materials were used in a similar manner to Step 2 of Example 26 to obtain 3-(3-cyanophenyl)prop-2-yn-1-yl methanesulfonate (0.703 g, 61.2%) in the form of a yellow solid: LRMS (ES) *m*/*z* no detection [M+H]⁺, calculated MW 235.26.

### Step 3: Synthesis of 3-(3-(4-(5-(difluoromethyl)-1,3,4-oxadiazol-2-yl)-2-oxopyridin-1(2H)-yl)prop-1-yn-1-yl)benzonitrile

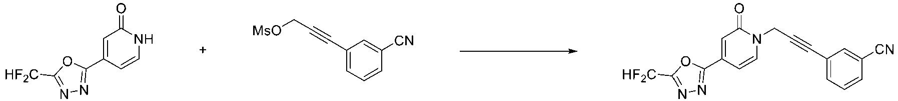

4-(5-(Difluoromethyl)-1,3,4-oxadiazol-2-yl)pyridin-2(1*H*)-one (0.100 g, 0.469 mmol), 3-(3-cyanophenyl)prop-2-yn-1-yl methanesulfonate (0.110 g, 0.469 mmol) and cesium carbonate (Cs₂CO₃, 0.459 g, 1.408 mmol) as starting materials were used in a similar manner to Step 3 of Example 26 to obtain 3-(3-(4-(5-(difluoromethyl)-1,3,4-oxadiazol-2-yl)-2-oxopyridin-1(2*H*)-yl)prop-1-yn-1-yl)benzonitrile (0.027 g, 16.3%) in the form of a yellow solid: LRMS (ES) *m*/*z* 353.14 [M+H]⁺, calculated MW 352.3; **¹H-NMR** (400 MHz, CDCl₃) d 7.82 (d, *J =* 6.8 Hz, 1 H), 7.74 (t, *J =* 1.4 Hz, 1 H), 7.69 ~ 7.63 (m, 2 H), 7.46 (t, *J =* 7.8 Hz, 1 H), 7.30 (d, *J =* 2.0 Hz, 2 H), 6.95 (dd, *J =* 7.4, 1.8 Hz, 1 H), 6.92 (t, *J =* 51.6 Hz, 1 H), 5.03 (s, 2 H).

### Example 32: Synthesis of 4-(5-(difluoromethyl)-1,3,4-oxadiazol-2-yl)-1-(3-(3-(methylsulfonyl)phenyl)prop-2-yn-1-yl)pyridin-2(1H)-one

### Step 1: Synthesis of 3-(3-(methylsulfonyl)phenyl)prop-2-yn-1-ol

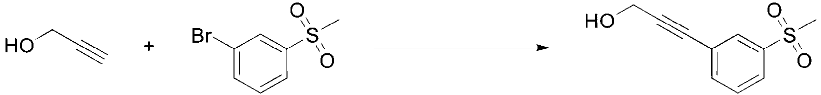

Prop-2-yn-1-ol (0.312 mL, 5.351 mmol), 1-bromo-3-(methylsulfonyl)benzene (1.258 g, 5.351 mmol), bis(triphenylphosphine)palladium(II) dichloride (0.075 g, 0.107 mmol), copper iodide (CuI, 0.020 g, 0.107 mmol) and triethylamine (2.238 mL, 16.054 mmol) as starting materials were used in a similar manner to Step 1 of Example 26 to obtain 3-(3-(methylsulfonyl)phenyl)prop-2-yn-1-ol (0.985 g, 87.5%) in the form of a yellow oil: **LRMS** (ES) *m*/*z* no detection [M+H]⁺, calculated MW 210.25.

### Step 2: Synthesis of 3-(3-(methylsulfonyl)phenyl)prop-2-yn-1-yl methanesulfonate

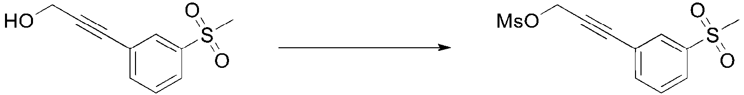

3-(3-(methylsulfonyl)phenyl)prop-2-yn-1-ol (0.985 g, 4.685 mmol), triethylamine (1.959 mL, 14.055 mmol) and methanesulfonyl chloride (0.725 mL, 9.370 mmol) as starting materials were used in a similar manner to Step 2 of Example 26 to obtain 3-(3-(methylsulfonyl)phenyl)prop-2-yn-1-yl methanesulfonate (0.812 g, 60.1%) in the form of a yellow solid: **LRMS** (ES) *m*/*z* no detection [M+H]⁺, calculated MW 288.33.

### Step 3: Synthesis of 4-(5-(difluoromethyl)-1,3,4-oxadiazol-2-yl)-1-(3-(3-(methylsulfonyl)phenyl)prop-2-yn-1-yl)pyridin-2(1H)-one

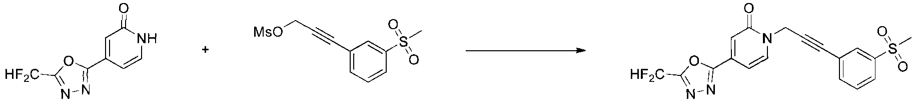

4-(5-(Difluoromethyl)-1,3,4-oxadiazol-2-yl)pyridin-2(1*H*)-one (0.100 g, 0.469 mmol) and 3-(3-(methylsulfonyl)phenyl)prop-2-yn-1-yl methanesulfonate as starting materials were used in a similar manner to Step 3 of Example 26 to obtain 4-(5-(difluoromethyl)-1,3,4-oxadiazol-2-yl)-1-(3-(3-(methylsulfonyl)phenyl)prop-2-yn-1-yl)pyridin-2(1H)-one (0.029 g, 15.2%) in the form of a yellow oil: **LRMS** (ES) *m*/*z* 406.05 [M+H]⁺, calculated MW 405.38; **¹H-NMR** (400 MHz, CDCl₃) d 8.04 (s, 1 H), 7.94 ~ 7.91 (m, 1 H), 7.84 (d, *J =* 7.2 Hz, 1 H), 7.72 (d, *J =* 7.6 Hz, 1 H), 7.56 (t, *J =* 8.0 Hz, 1 H), 7.30 (d, *J =* 2.0 Hz, 1 H), 6.96 (dd, *J =* 7.2, 2.0 Hz, 1 H), 6.92 (t, *J =* 51.6 Hz, 1 H), 5.04 (s, 2 H), 3.05 (s, 3 H).

### Example 33: Synthesis of 1-(3-(3-chloro-2-fluorophenyl)prop-2-yn-1-yl)-4-(5-(difluoromethyl)-1,3,4-oxadiazol-2-yl)pyridin-2(1H)-one

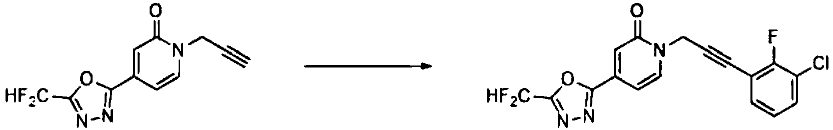

4-(5-(Difluoromethyl)-1,3,4-oxadiazol-2-yl)-1-(prop-2-yn-1-yl)pyridin-2(1*H*)-one (0.100 g, 0.398 mmol), triethylamine (0.277 mL, 1.991 mmol), bis(triphenylphosphine)palladium(II) dichloride (0.014 g, 0.020 mmol), copper iodide (CuI, 0.009 g, 0.048 mmol) and 1-chloro-2-fluoro-3-iodobenzene (0.112 g, 0.438 mmol) as starting materials were used in a similar manner to Step 4 of Example 2 to obtain 1-(3-(3-chloro-2-fluorophenyl)prop-2-yn-1-yl)-4-(5-(difluoromethyl)-1,3,4-oxadiazol-2-yl)pyridin-2(1*H*)-one (0.080 g, 52.9%) in the form of a yellow solid: **LRMS** (ES) *m*/*z* 380.02 [M+H]⁺, calculated MW 379.72; 1H-NMR (400 MHz, DMSO-*d*₆) d 8.08 (d, *J =* 6.8 Hz, 1 H), 7.52 (t, *J* = 51.2 Hz, 1 H), 7.62 (td, *J =* 7.7, 1.5 Hz, 1 H), 7.49 ~ 7.48 (m, 1 H), 7.21 (td, *J =* 8.0, 0.8 Hz, 1 H), 7.02 (d, *J =* 1.6 Hz, 1 H), 6.84 (dd, *J* = 7.0*,* 1.8 Hz, 1 H), 5.10 (s, 2 H).

### Example 34: Synthesis of 1-(3-(3-chloro-4-fluorophenyl)prop-2-yn-1-yl)-4-(5-(difluoromethyl)-1,3,4-oxadiazol-2-yl)pyridin-2(1H)-one

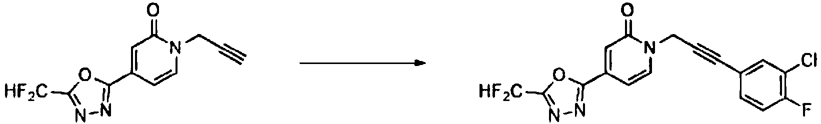

4-(5-(Difluoromethyl)-1,3,4-oxadiazol-2-yl)-1-(prop-2-yn-1-yl)pyridin-2(1*H*)-one (0.100 g, 0.398 mmol), triethylamine (0.277 mL, 1.991 mmol), bis(triphenylphosphine)palladium(II) dichloride (0.014 g, 0.020 mmol), copper iodide (CuI, 0.004 g, 0.020 mmol) and 2-chloro-1-fluoro-4-iodobenzene (0.112 g, 0.438 mmol) as starting materials were used in a similar manner to Step 4 of Example 2 to obtain 1-(3-(3-chloro-4-fluorophenyl)prop-2-yn-1-yl)-4-(5-(difluoromethyl)-1,3,4-oxadiazol-2-yl)pyridin-2(1H)-one (0.092 g, 60.9%) in the form of a yellow solid: **LRMS** (ES) *m*/*z* 380.09 [M+H]⁺, calculated MW 379.72; 1H-NMR (400 MHz, DMSO-*d*₆) d 8.09 (d, *J =* 7.2 Hz, 1 H), 7.72 (dd, *J =* 7.0, 1.8 Hz, 1 H), 7.52 (t, *J* = 51.2 Hz, 1 H), 7.47 ~ 7.46 (m, 1 H), 7.43 ~ 7.39 (m, 1 H), 7.01 (d, *J =* 2.0 Hz, 1 H), 6.83 (dd, *J =* 7.2, 2.0 Hz, 1 H), 5.03 (s, 2 H).

### Example 35: Synthesis of 1-(3-(3-chloro-5-fluorophenyl)prop-2-yn-1-yl)-4-(5-(difluoromethyl)-1,3,4-oxadiazol-2-yl)pyridin-2(1H)-one

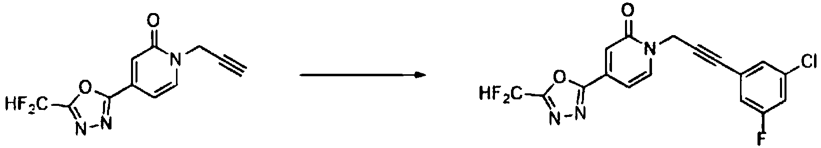

4-(5-(Difluoromethyl)-1,3,4-oxadiazol-2-yl)-1-(prop-2-yn-1-yl)pyridin-2(1*H*)-one (0.100 g, 0.398 mmol), triethylamine (0.277 mL, 1.991 mmol), bis(triphenylphosphine)palladium(II) dichloride (0.014 g, 0.020 mmol), copper iodide (CuI, 0.004 g, 0.020 mmol) and 1-chloro-3-fluoro-5-iodobenzene (0.112 g, 0.438 mmol) as starting materials were used in a similar manner to Step 4 of Example 2 to obtain 1-(3-(3-chloro-5-fluorophenyl)prop-2-yn-1-yl)-4-(5-(difluoromethyl)-1,3,4-oxadiazol-2-yl)pyridin-2(1H)-one (0.083 g, 54.9%) in the form of a yellow solid: **LRMS** (ES) *m*/*z* 380.09 [M+H]⁺, calculated MW 379.72; 1H-NMR (400 MHz, DMSO-*d*₆) d 8.09 (d, *J =* 7.6 Hz, 1 H), 7.52 (t, *J* = 51.2 Hz, 1 H), 7.50 (td, *J =* 5.5, 2.9 Hz, 1 H), 7.41 (s, 1 H), 7.37 ~ 7.34 (m, 1 H), 7.01 (d, *J =* 1.2 Hz, 1 H), 6.83 (dd, *J =* 7.0, 1.8 Hz, 1 H), 5.05 (s, 2 H).

### Example 36: Synthesis of 1-(3-(5-chloro-2-fluorophenyl)prop-2-yn-1-yl)-4-(5-(difluoromethyl)-1,3,4-oxadiazol-2-yl)pyridin-2(1H)-one

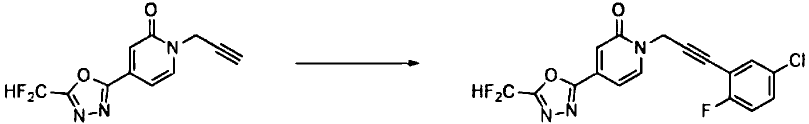

4-(5-(Difluoromethyl)-1,3,4-oxadiazol-2-yl)-1-(prop-2-yn-1-yl)pyridin-2(1*H*)-one (0.100 g, 0.398 mmol), triethylamine (0.277 mL, 1.991 mmol), bis(triphenylphosphine)palladium(II) dichloride (0.014 g, 0.020 mmol), copper iodide (CuI, 0.004 g, 0.020 mmol) and 4-chloro-1-fluoro-2-iodobenzene (0.112 g, 0.438 mmol) as starting materials were used in a similar manner to Step 4 of Example 2 to obtain 1-(3-(5-chloro-2-fluorophenyl)prop-2-yn-1-yl)-4-(5-(difluoromethyl)-1,3,4-oxadiazol-2-yl)pyridin-2(1*H*)-one (0.095 g, 62.8%) in the form of a yellow solid: **LRMS** (ES) *m*/*z* 380.09 [M+H]⁺, calculated MW 379.72; **¹H-NMR**(400 MHz, DMSO-*d*₆) d 8.08 (d, *J =* 7.2 Hz, 1 H), 7.52 (t, *J* = 51.2 Hz, 1 H), 7.63 (dd, *J* = 5.8, 2.6 Hz, 1 H), 7.50 ~ 7.49 (m, 1 H), 7.34 (t, *J =* 9.2 Hz, 1 H), 7.02 (d, *J =* 1.6 Hz, 1 H), 6.84 (dd, *J =* 7.0*,* 1.8 Hz, 1 H), 5.09 (s, 2 H).

### Example 37: Synthesis of 2-(3-(3-chlorophenyl)prop-2-yn-1-yl)-5-(5-(difluoromethyl)-1,3,4-oxadiazol-2-yl)pyridazin-3(2H)-one

### Step 1: Synthesis of 6-oxo-1,6-dihydropyridazine-4-carbohydrazide

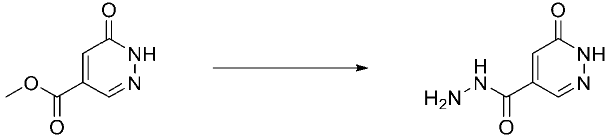

A solution in which methyl 6-oxo-1,6-dihydropyridazine-4-carboxylate (1.000 g, 6.488 mmol) was dissolved in ethanol (50 mL) at room temperature, and hydrazine hydrate (1.532 mL, 32.440 mmol) as starting materials were used in a similar manner to Step 1 of Example 8 to obtain 6-oxo-1,6-dihydropyridazine-4-carbohydrazide (0.966 g, 96.6%) in the form of a yellow solid: **LRMS** (ES) *m*/*z* 155.33 [M+H]⁺, calculated MW 154.13.

### Step 2: Synthesis of 5-(5-(difluoromethyl)-1,3,4-oxadiazol-2-yl)pyridazin-3 (2H)-one

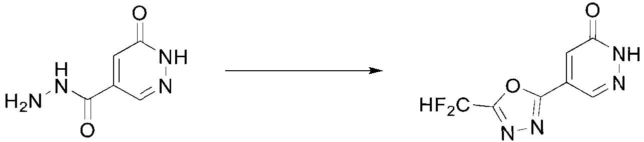

6-Oxo-1,6-dihydropyridazine-4-carbohydrazide (0.966 g, 6.267 mmol), imidazole (1.280 g, 18.802 mmol) and 2,2-difluoroacetic anhydride (2.338 mL, 18.802 mmol) as starting materials were used in a similar manner to Step 2 of Example 8 to obtain 5-(5-(difluoromethyl)-1,3,4-oxadiazol-2-yl)pyridazin-3(2H)-one (0.435 g, 32.4%) in the form of a yellow solid: **LRMS** (ES) *m*/*z* 215.80 [M+H]⁺, calculated MW 214.13.

### Step 3: Synthesis of 2-(3-(3-chlorophenyl)prop-2-yn-1-yl)-5-(5-(difluoromethyl)-1,3,4-oxadiazol-2-yl)pyridazin-3(2H)-one

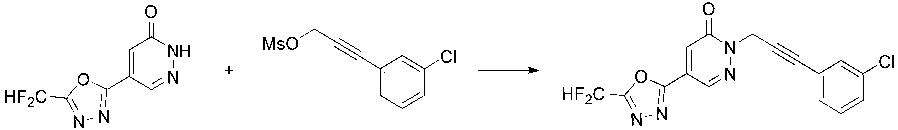

5-(5-(Difluoromethyl)-1,3,4-oxadiazol-2-yl)pyridazin-3(2*H*)-one (0.100 g, 0.467 mmol), 3-(3-chlorophenyl)prop-2-yn-1-yl methanesulfonate (0.120 g, 0.490 mmol) and cesium carbonate (Cs₂CO₃, 0.228 g, 0.701 mmol) as starting materials were used in a similar manner to Step 3 of Example 26 to obtain 2-(3-(3-chlorophenyl)prop-2-yn-1-yl)-5-(5-(difluoromethyl)-1,3,4-oxadiazol-2-yl)pyridazin-3(2H)-one (0.032 g, 18.9%) in the form of a light brown solid: **LRMS** (ES) *m*/*z* no detection [M+H]⁺, calculated MW 362.72; **¹H-NMR** (400 MHz, DMSO-*d*₆) d 7.74 (s, 1 H), 7.54 (t, *J =* 1.6 Hz, 1 H), 7.47 ~ 7.38 (m, 3 H), 7.27 (s, 1 H), 6.91 (s, 1 H), 5.16 (s, 2 H).

### Example 38: Synthesis of 1-(3-(3-chlorophenyl)prop-2-yn-1-yl)-4-(5-(difluoromethyl)-1,3,4-oxadiazol-2-yl)pyrimidin-2(1H)-one

### Step 1: Synthesis of 2-oxo-1,2-dihydropyrimidine-4-carbohydrazide

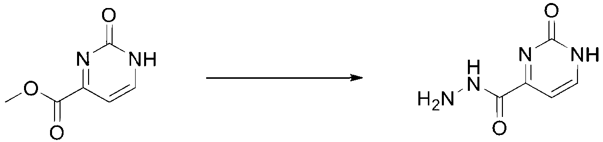

A solution in which methyl 2-oxo-1,2-dihydropyrimidine-4-carboxylate (0.500 g, 3.244 mmol) was dissolved in ethanol (20 mL) at room temperature, and hydrazine hydrate (0.766 mL, 16.220 mmol) as starting materials were used in a similar manner to Step 1 of Example 8 to obtain 2-oxo-1,2-dihydropyrimidine-4-carbohydrazide (0.494 g, 98.8%) in the form of a yellow liquid: **LRMS** (ES) *m*/*z* 155.04 [M+H]⁺, calculated MW 154.13.

### Step 2: Synthesis of 4-(5-(difluoromethyl)-1,3,4-oxadiazol-2-yl)pyrimidin-2(1H)-one

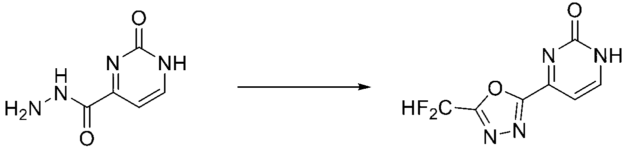

2-Oxo-1,2-dihydropyrimidine-4-carbohydrazide (0.570 g, 3.698 mmol) and imidazole (0.755 g, 11.095 mmol) were dissolved in dichloromethane (10 mL) and stirred at room temperature for 30 minutes. The obtained solution and 2,2-difluoroacetic anhydride (1.379 mL, 11.095 mmol) as starting materials were used in a similar manner to Step 2 of Example 8 to obtain 4-(5-(difluoromethyl)-1,3,4-oxadiazol-2-yl)pyrimidin-2(1*H*)-one (0.710 g, 89.7%) in the form of a yellow solid: **LRMS** (ES) *m*/*z* no detection [M+H]⁺, calculated MW 214.13.

### Step 3: Synthesis of 1-(3-(3-chlorophenyl)prop-2-yn-1-yl)-4-(5-(difluoromethyl)-1,3,4-oxadiazol-2-yl)pyrimidin-2(1H)-one

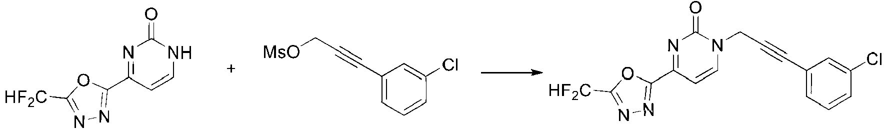

4-(5-(Difluoromethyl)-1,3,4-oxadiazol-2-yl)pyrimidin-2(1*H*)-one (0.100 g, 0.467 mmol), 3-(3-chlorophenyl)prop-2-yn-1-yl methanesulfonate (0.120 g, 0.490 mmol) and cesium carbonate (Cs₂CO₃, 0.228 g, 0.701 mmol) as starting materials were used in a similar manner to Step 3 of Example 26 to obtain 1-(3-(3-chlorophenyl)prop-2-yn-1-yl)-4-(5-(difluoromethyl)-1,3,4-oxadiazol-2-yl)pyrimidin-2(1*H*)-one (0.024 g, 14.2%) in the form of a light brown solid: **LRMS** (ES) *m*/*z* no detection [M+H]⁺, calculated MW 362.72; **¹H-NMR** (400 MHz, DMSO-*d*₆) d 7.73 (s, 1 H), 7.53 ~ 7.53 (m, 1 H), 7.47 ~ 7.36 (m, 3 H), 7.27 (s, 1 H), 6.91 (s, 1 H), 5.16 (s, 2 H).

### Example 39: Synthesis of 3-(3-(3-chlorophenyl)prop-2-yn-1-yl)-6-(5-(difluoromethyl)-1,3,4-oxadiazol-2-yl)pyrimidin-4(3H)-one

### Step 1: Synthesis of 6-oxo-1,6-dihydropyrimidine-4-carbohydrazide

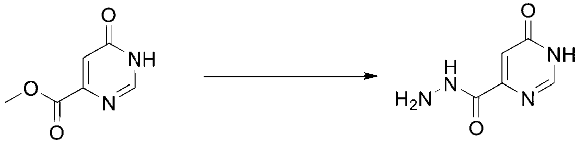

A solution in which methyl 6-oxo-1,6-dihydropyrimidine-4-carboxylate (1.000 g, 6.488 mmol) was dissolved in ethanol (50 mL) at room temperature, and hydrazine hydrate (1.624 g, 32.440 mmol) as starting materials were used in a similar manner to Step 1 of Example 8 to obtain 6-oxo-1,6-dihydropyrimidine-4-carbohydrazide (0.996 g, 99.6%) in the form of a white solid: **LRMS** (ES) *m*/*z* 155.30 [M+H]⁺, calculated MW 154.13.

### Step 2: Synthesis of 6-(5-(difluoromethyl)-1,3,4-oxadiazol-2-yl)pyrimidin-4(3H)-one

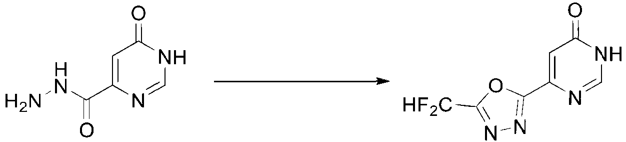

6-Oxo-1,6-dihydropyrimidine-4-carbohydrazide (0.996 g, 6.462 mmol), imidazole (1.320 g, 19.386 mmol) and 2,2-difluoroacetic anhydride (2.410 mL, 19.386 mmol) as starting materials were used in a similar manner to Step 2 of Example 8 to obtain 6-(5-(difluoromethyl)-1,3,4-oxadiazol-2-yl)pyrimidine-4(3*H*)-one (0.231 g, 16.7%) was obtained as a brown liquid: **LRMS** (ES) *m*/*z* 215.08 [M+H]⁺, calculated MW 214.13.

### Step 3: Synthesis of 3-(3-(3-chlorophenyl)prop-2-yn-1-yl)-6-(5-(difluoromethyl)-1,3,4-oxadiazol-2-yl)pyrimidin-4(3H)-one

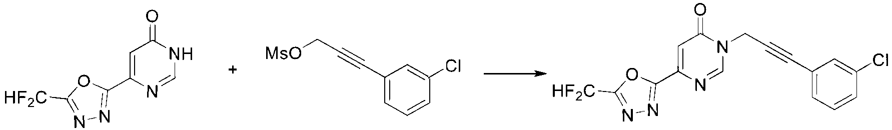

6-(5-(Difluoromethyl)-1,3,4-oxadiazol-2-yl)pyrimidin-4(3*H*)-one (0.100 g, 0.467 mmol), 3-(3-chlorophenyl)prop-2-yn-1-yl methanesulfonate (0.120 g, 0.490 mmol) and cesium carbonate (Cs₂CO₃, 0.228 g, 0.701 mmol) as starting materials were used in a similar manner to Step 3 of Example 26 to obtain 3-(3-(3-chlorophenyl)prop-2-yn-1-yl)-6-(5-(difluoromethyl)-1,3,4-oxadiazol-2-yl)pyrimidin-4(3H)-one (0.021 g, 12.4%) in the form of a brown solid: **LRMS** (ES) *m*/*z* no detection [M+H]⁺, calculated MW 362.72; **¹H-NMR** (400 MHz, DMSO-*d*₆) d 7.74 (s, 1 H), 7.53 ~ 7.53 (m, 1 H), 7.47 ~ 7.36 (m, 3 H), 7.27 (s, 1 H), 6.92 (s, 1 H), 5.16 (s, 2 H).

### Example 40: Synthesis of 1-(3-(benzo[d]thiazol-5-yl)prop-2-yn-1-yl)-4-(5-(difluoromethyl)-1,3,4-oxadiazol-2-yl)pyridin-2(1H)-one

### Step 1: Synthesis of 3-(benzo[d]thiazol-5-yl)prop-2-yn-1-ol

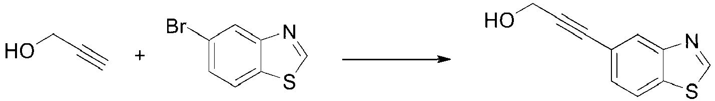

Prop-2-yn-1-ol (0.100 g, 1.784 mmol), 5-bromobenzo[d]thiazole (0.401 g, 1.873 mmol), tetrakis(triphenylphosphine)palladium (0.103 g, 0.089 mmol), copper iodide (CuI, 0.017 g, 0.089 mmol) and triethylamine (0.746 mL, 5.351 mmol) were mixed with *N,N-*dimethylformamide (4 mL) at room temperature. The obtained mixture was irradiated with microwaves and heated at 120°C for 30 minutes, and the temperature was lowered to room temperature to terminate the reaction. The solvent was removed from the reaction mixture under reduced pressure, and the obtained concentrate was poured with a saturated aqueous ammonium chloride solution and extracted with ethyl acetate. The organic layer was washed with saturated aqueous sodium chloride solution, dried over anhydrous magnesium sulfate, filtered and concentrated under reduced pressure. The concentrate was purified and concentrated by column chromatography to obtain 3-(benzo[d]thiazol-5-yl)prop-2-yn-1-ol (0.052 g, 15.4%) in the form of a yellow solid: **LRMS** (ES) *m*/*z* 190.21 [M+H]⁺, calculated MW 189.23.

### Step 2: Synthesis of 1-(3-(benzo[d]thiazol-5-yl)prop-2-yn-1-yl)-4-(5-(difluoromethyl)-1,3,4-oxadiazol-2-yl)pyridin-2(1H)-one

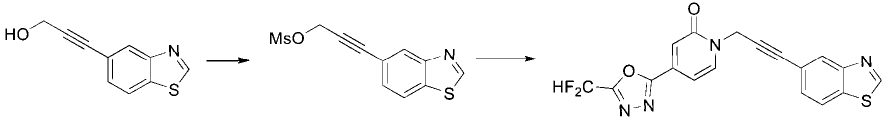

3-(Benzo[d]thiazol-5-yl)prop-2-yn-1-ol (0.052 g, 0.275 mmol), triethylamine (0.046 mL, 0.330 mmol), 4-(5-(difluoromethyl )-1,3,4-oxadiazol-2-yl)pyridin-2(1*H*)-one (0.070 g, 0.330 mmol) and cesium carbonate (Cs₂CO₃, 0.134 g, 0.412 mmol) as starting materials were used in a similar manner to Step 2 of Example 29 to obtain 1-(3-(benzo[*d*]thiazol-5-yl)prop-2-yn-1-yl)-4-(5-(difluoromethyl)-1,3,4-oxadiazol-2-yl)pyridin-2(1*H*)-one (0.016 g, 15.1%) in the form of a brown solid: **LRMS** (ES) *m*/*z* 385.18 [M+H]⁺, calculated MW 384.36; **¹H-NMR** (400 MHz, DMSO-*d*₆) d 9.43 (s, 1 H), 8.19 ~ 8.14 (m, 3 H), 7.66 ~ 7.40 (m, 1 H), 7.53 (s, 1 H), 7.02 (d, *J* = 2.0 Hz, 1 H), 6.85 (dd, *J =* 7.0, 2.2 Hz, 1 H), 5.08 (s, 2 H).

### Example 41: Synthesis of 4-(5-(difluoromethyl)-1,3,4-oxadiazol-2-yl)-1-(3-(2-methylbenzo[d]thiazol-6-yl)prop-2-yn-1-yl)pyridin-2(1H)-one

### Step 1: Synthesis of 3-(2-methylbenzo[d]lthiazol-6-yl)prop-2-yn-1-ol

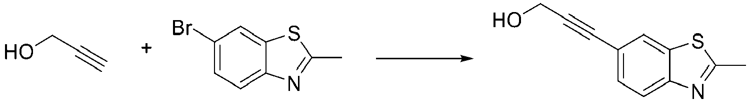

Prop-2-yn-1-ol (0.025 g, 0.446 mmol), 6-bromo-2-methylbenzo[d]thiazole (0.107 g, 0.468 mmol), tetrakis(triphenylphosphine)palladium (0.026 g, 0.022 mmol), copper iodide (CuI, 0.004 g, 0.022 mmol) and triethylamine (0.186 mL, 1.338 mmol) were used in a similar manner to Step 1 of Example 40 to obtain 3-(2-methylbenzo[d]thiazol-6-yl)prop-2-yn-1-ol (0.068 g, 75.0%) in the form of a brown liquid: **LRMS** (ES) *m*/*z* 204.05 [M+H]⁺, calculated MW 203.26.

### Step 2: Synthesis of 4-(5-(difluoromethyl)-1,3,4-oxadiazol-2-yl)-1-(3-(2-methylbenzo[d]thiazol-6-yl)prop-2-yn-1-yl)pyridin-2(1H)-one

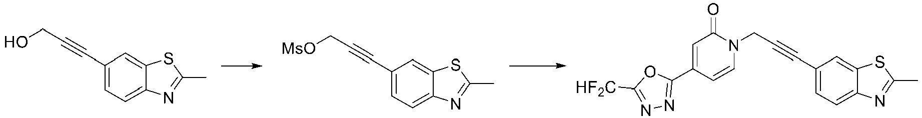

3-(2-Methylbenzo[*d*]thiazol-6-yl)prop-2-yn-1-ol (0.060 g, 0.295 mmol), triethylamine (0.049 mL, 0.354 mmol), methanesulfonyl chloride (0.027 mL, 0.354 mmol), 4-(5-(difluoromethyl)-1,3,4-oxadiazol-2-yl)pyridin-2(1*H*)-one (0.075 g, 0.354 mmol) and cesium carbonate (Cs₂CO₃, 0.144 g, 0.443 mmol) as starting materials were used in a similar manner to Step 2 of Example 29 to obtain 4-(5-(difluoromethyl)-1,3,4-oxadiazol-2-yl)-1-(3-(2-methylbenzo[*d*]thiazol-6-yl)prop-2-yn-1-yl)pyridin-2(1*H*)-one (0.005 g, 4.3%) in the form of a brown solid: **LRMS** (ES) *m*/*z* 399.18 [M+H]⁺, calculated MW 398.39; **¹H-NMR** (400 MHz, DMSO-*d*₆) d 8.43 (d, *J =* 5.2 Hz, 1 H), 8.00 (d, *J =* 2.0 Hz, 1 H), 7.81 (d, *J =* 8.4 Hz, 1 H), 7.63 (dd, *J =* 5.4, 1.4 Hz, 1 H), 7.52 ~ 7.49 (m, 2 H), 7.23 (t, *J* = 51.8 Hz, 1 H), 5.29 (s, 2 H), 2.80 (s, 3 H).

### Example 42: Synthesis of 4-(5-(difluoromethyl)-1,3,4-oxadiazol-2-yl)-1-(3-(2-methyl-1H-imidazol-5-yl)prop-2-yn-1-yl)pyridin-2(1H)-one

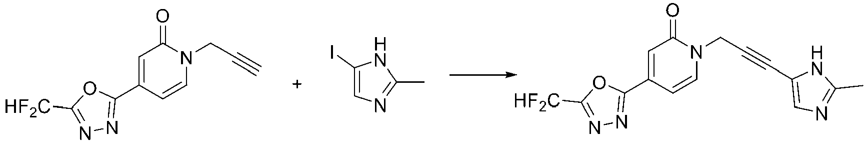

5-Iodo-2-methyl-1*H*-imidazole (0.050 g, 0.240 mmol), 4-(5-(difluoromethyl)-1,3,4-oxadiazol-2-yl)-1-(prop-2-yn-1-yl)pyridin-2(1*H*)-one (0.078 g, 0.313 mmol), copper iodide (CuI, 0.005 g, 0.024 mmol), 1,1'-bis(diphenylphosphino)ferrocene (0.013 g, 0.024 mmol) and methanesulfonato 1,1-ferrocenediyl-bis(diphenylphosphino)(2'-amino-1,1'-biphenyl-2-yl)palladium(II) (0.022 g, 0.024 mmol) as starting materials were used in a similar manner to Step 4 of Example 2 to obtain 4-(5-(difluoromethyl)-1,3,4-oxadiazol-2-yl)-1-(3-(2-methyl-1*H*-imidazol-5-yl)prop-2-yn-1-yl)pyridin-2(1*H*)-one (0.022 g, 27.6%) in the form of a brown solid: **LRMS** (ES) *m*/*z* 332.20 [M+H]⁺, calculated MW 331.28; **¹H-NMR** (400 MHz, DMSO-*d*₆) d 11.92 (s, 1 H), 8.04 (d, *J =* 6.8 Hz, 1 H), 7.65 ~ 7.29 (m, 2 H), 6.99 (d, *J =* 2.0 Hz, 1 H), 6.83 (dd, *J =* 6.8, 1.6 Hz, 1 H), 4.96 (s, 2 H), 2.18 (s, 3 H).

### Example 43: Synthesis of 4-(5-(difluoromethyl)-1,3,4-oxadiazol-2-yl)-1-(3-(thiophen-3-yl)prop-2-yn-1-yl)pyridin-2(1H)-one

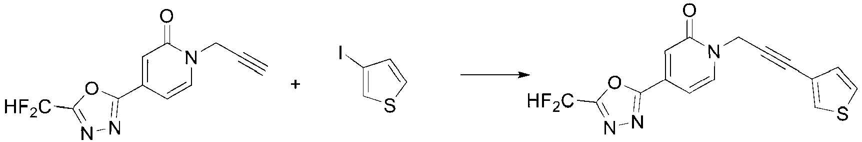

3-Iodothiophene (0.030 g, 0.143 mmol), 4-(5-(difluoromethyl)-1,3,4-oxadiazol-2-yl)-1-(prop-2-yn-1-yl)pyridin-2(1*H*)-one (0.047 g, 0.186 mmol), copper iodide (CuI, 0.003 g, 0.014 mmol), 1,1'-ferrocenedinyl-bis(diphenylphosphine) (0.008 g, 0.014 mmol) and methanesulfonato 1,1-ferrocenediyl-bis(diphenylphosphino)(2'-amino-1,1'-biphenyl-2-yl)palladium(II) (0.013 g, 0.014 mmol) as starting materials were used in a similar manner to Step 4 of Example 2 to obtain 4-(5-(difluoromethyl)-1,3,4-oxadiazol-2-yl)-1-(3-(thiophen-3-yl)prop-2-yn-1-yl)pyridin-2(1H)-one (0.017 g, 36.5%) in the form of a brown solid: **LRMS** (ES) *m*/*z* 334.12 [M+H]⁺, calculated MW 333.31; **¹H-NMR** (400 MHz, DMSO-*d*₆) d 8.07 (d, *J =* 7.2 Hz, 1 H), 7.81 (dd, *J* = 3.0, 1.0 Hz, 1 H), 7.65 ~ 7.40 (m, 1 H), 7.59 ~ 7.57 (m, 1 H), 7.14 (dd, *J =* 5.2, 1.2 Hz, 1 H), 7.00 (d, *J =* 2.0 Hz, 1 H), 6.83 (dd, *J = 7.2,* 2.0 Hz, 1 H), 5.00 (s, 2 H).

### Example 44: Synthesis of 1-(1-(3-chlorophenyl)pent-1-yn-3-yl)-4-(5-(difluoromethyl)-1,3,4-oxadiazol-2-yl)pyridin-2(1H)-one

### Step 1: Synthesis of 1-(3-chlorophenyl)pent-1-yn-3-ol

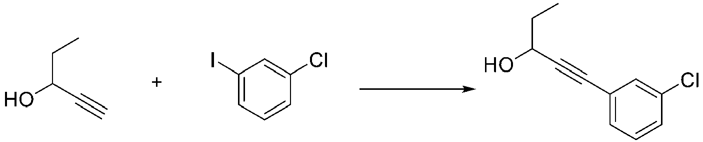

1-Chloro-3-iodobenzene (0.500 g, 2.097 mmol), pent-1-yn-3-ol (0.229 g, 2.726 mmol), tetrakis(triphenylphosphine)palladium (0.121 g, 0.105 mmol), copper iodide (CuI, 0.020 g, 0.105 mmol) and triethylamine (0.877 mL, 6.291 mmol) as starting materials were used in a similar manner to Step 1 of Example 40 to obtain 1-(3-chlorophenyl)pent-1-yn-3-ol (0.190 g, 46.5%) in the form of a yellow liquid: **LRMS** (ES) *m*/*z* no detection [M+H]⁺, calculated MW 194.66; **¹H-NMR** (400 MHz, DMSO-*d*₆) d 7.42 ~ 7.39 (m, 2 H), 7.38 ~ 7.32 (m, 2 H), 5.44 (d, *J* = 5.6 Hz, 1 H), 4.34 (q, *J =* 6.0 Hz, 1 H), 1.67 ~ 1.56 (m, 2 H), 0.93 (t, *J* = 7.4 Hz, 3 H).

### Step 2: Synthesis of 1-(3-bromopent-1-yn-1-yl)-3-chlorobenzene

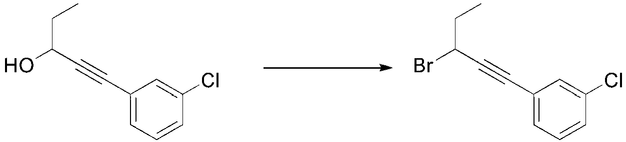

1-(3-Chlorophenyl)pent-1-yn-3-ol (0.200 g, 1.027 mmol) was dissolved in dichloromethane (4 mL), and phosphorus tribromide (0.105 mL, 1.110 mmol) was added thereto at 0°C and stirred at the same temperature for 1 hour and additionally stirred at room temperature for 3 hours. Water (0.555 mL, 30.823 mmol) was added to the reaction mixture at room temperature and stirred for 5 minutes to terminate the reaction. A saturated aqueous solution of sodium hydrogen carbonate was poured into the reaction mixture, and the obtained mixture was extracted with dichloromethane. The organic layer was washed with saturated aqueous sodium chloride solution, dried over anhydrous magnesium sulfate, filtered and concentrated under reduced pressure. The concentrate was purified and concentrated by column chromatography to obtain 1-(3-bromopent-1-yn-1-yl)-3-chlorobenzene (0.165 g, 62.4%) in the form of a brown solid: R_{f} 0.4; **LRMS** (ES) *m*/*z* no detection [M+H]⁺, calculated MW 257.56.

### Step 3: Synthesis of 1-(1-(3-chlorophenyl)pent-1-yn-3-yl)-4-(5-(difluoromethyl)-1,3,4-oxadiazol-2-yl)pyridin-2(1H)-one

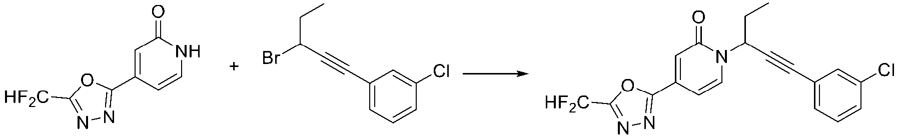

4-(5-(Difluoromethyl)-1,3,4-oxadiazol-2-yl)pyridin-2(1*H*)-one (0.050 g, 0.235 mmol), 1-(3-bromopent-1-yn-1-yl)-3-chlorobenzene (0.066 g, 0.258 mmol) and potassium carbonate (0.097 g, 0.704 mmol) as starting materials were used in a similar manner to Step 3 of Example 26 to obtain 1-(1-(3-chlorophenyl)pent-1-yn-3-yl)-4-(5-(difluoromethyl)-1,3,4-oxadiazol-2-yl)pyridin-2(1*H*)-one (0.029 g, 31.7%) in the form of a brown solid: **LRMS** (ES) *m*/*z* 390.20 [M+H]⁺, calculated MW 389.79; **¹H-NMR** (400 MHz, DMSO-*d*₆) d 8.46 (d, *J =* 6.0 Hz, 1 H), 7.67 ~ 7.54 (m, 2 H), 7.45 ~ 7.40 (m, 3 H), 7.36 ~ 7.33 (m, 2 H), 5.88 (t, *J =* 6.4 Hz, 1 H), 2.00 ~ 1.93 (m, 2 H), 1.07 (t, *J =* 7.2 Hz, 3 H).

### Example 45: Synthesis of 1-(3-(3-chlorophenyl)prop-2-yn-1-yl)-4-(5-(difluoromethyl)-1,3,4-oxadiazol-2-yl)-6-methylpyridin-2(1H)-one

### Step 1: Synthesis of 6-methyl-2-oxo-1,2-dihydropyridine-4-carbohydrazide

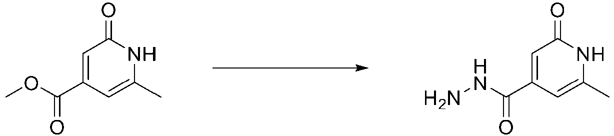

Methyl 6-methyl-2-oxo-1,2-dihydropyridine-4-carboxylate (2.000 g, 11.965 mmol) and hydrazine monohydrate (0.756 mL, 15.554 mmol) as starting materials were used in a similar manner to Step 1 of Example 8 to obtain 6-methyl-2-oxo-1,2-dihydropyridine-4-carbohydrazide (1.731 g, 86.5%) in the form of a beige solid: **LRMS** (ES) *m*/*z* 168.05 [M+H]⁺, calculated MW 167.17.

### Step 2: Synthesis of 4-(5-(difluoromethyl)-1,3,4-oxadiazol-2-yl)-6-methylpyridin-2(1H)-one

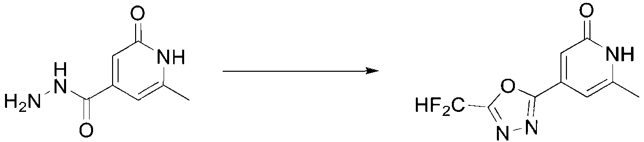

6-Methyl-2-oxo-1,2-dihydropyridine-4-carbohydrazide (1.731 g, 10.355 mmol), imidazole (1.719 mL, 31.064 mmol) and 2,2-difluoroacetic anhydride (2.832 mL, 22.780 mmol) as starting materials were used in a similar manner to Step 2 of Example 8 to obtain 4-(5-(difluoromethyl)-1,3,4-oxadiazol-2-yl)-6-methylpyridin-2(1*H*)-one (1.821 g, 77.4%) in the form of an ivory solid: **LRMS** (ES) *m*/*z* 228.16 [M+H]⁺, calculated MW 227.17.

### Step 3: Synthesis of 1-(3-(3-chlorophenyl)prop-2-yn-1-yl)-4-(5-(difluoromethyl)-1,3,4-oxadiazol-2-yl)-6-methylpyridin-2(1H)-one

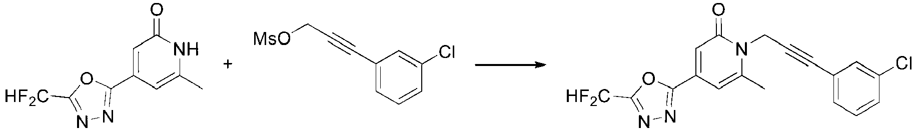

4-(5-(Difluoromethyl)-1,3,4-oxadiazol-2-yl)-6-methylpyridin-2(1*H*)-one (0.100 g, 0.440 mmol), 3-(3-chlorophenyl)prop-2-yn-1-yl methanesulfonate (0.118 g, 0.484 mmol) and potassium carbonate (0.122 g, 0.880 mmol) as starting materials were used in a similar manner to Step 3 of Example 26 to obtain 1-(3-(3-chlorophenyl)prop-2-yn-1-yl)-4-(5-(difluoromethyl)-1,3,4-oxadiazol-2-yl)-6-methylpyridin-2(1*H*)-one (0.036 g, 21.8%) in the form of a beige solid: **LRMS** (ES) *m*/*z* 376.19 [M+H]⁺, calculated MW 375.76; **¹H-NMR** (400 MHz, DMSO-*d*₆) d 7.51 (t, *J =* 52.2 Hz, 1 H), 7.52 (m, 1 H), 7.46 ~ 7.43 (m, 1 H), 7.39 ~ 7.36 (m, 2 H), 6.90 (s, 1 H), 6.78 (s, 1 H), 5.14 (s, 2 H), 2.63 (s, 3 H).

### Example 46: Synthesis of 5-bromo-1-(3-(3-chlorophenyl)prop-2-yn-1-yl)-4-(5-(difluoromethyl)-1,3,4-oxadiazol-2-yl)pyridin-2(1H)-one

### Step 1: Synthesis of 5-bromo-2-oxo-1,2-dihydropyridine-4-carbohydrazide

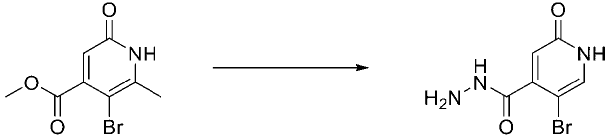

Methyl 5-bromo-2-oxo-1,2-dihydropyridine-4-carboxylate (2.000 g, 8.620 mmol) and hydrazine monohydrate (0.545 mL, 11.205 mmol) as starting materials were used in a similar manner to Step 1 of Example 8 to obtain 5-bromo-2-oxo-1,2-dihydropyridine-4-carbohydrazide (1.619 g, 80.9%) in the form of a beige solid: **LRMS** (ES) *m*/*z* 232.06/234.04 [M+H]⁺, calculated MW 232.04.

### Step 2: Synthesis of 5-bromo-4-(5-(difluoromethyl)-1,3,4-oxadiazol-2-yl)pyridin-2(1H)-one

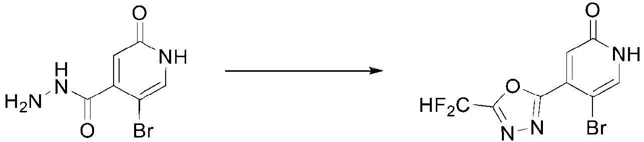

5-Bromo-2-oxo-1,2-dihydropyridine-4-carbohydrazide (1.619 g, 6.977 mmol), imidazole (1.159 mL, 20.932 mmol) and 2,2-difluoroacetic anhydride (1.908 mL, 15.350 mmol) as starting materials were used in a similar manner to Step 2 of Example 8 to obtain 5-bromo-4-(5-(difluoromethyl)-1,3,4-oxadiazole-2-yl)pyridin-2(1*H*)-one (0.589 g, 28.9%) in the form of a light yellow solid: **LRMS** (ES) *m*/*z* 292.04 [M+H]⁺, calculated MW 292.04.

### Step 3: Synthesis of 5-bromo-1-(3-(3-chlorophenyl)prop-2-yn-1-yl)-4-(5-(difluoromethyl)-1,3,4-oxadiazol-2-yl)pyridin-2(1H)-one

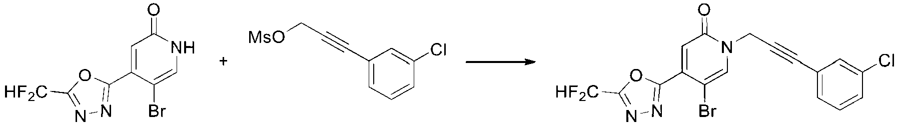

5-Bromo-4-(5-(difluoromethyl)-1,3,4-oxadiazol-2-yl)pyridin-2(1*H*)-one (0.100 g, 0.342 mmol), 3-(3-chlorophenyl)prop-2-yn-1-yl methanesulfonate (0.092 g, 0.377 mmol) and potassium carbonate (0.095 g, 0.685 mmol) as starting materials were used in a similar manner to Step 3 of Example 26 to obtain 5-bromo-1-(3-(3-chlorophenyl)prop-2-yn-1-yl)-4-(5-(difluoromethyl)-1,3,4-oxadiazol-2-yl)pyridin-2(1*H*)-one (0.027 g, 17.9%) in the form of a brown solid: **LRMS** (ES) *m*/*z* no detection [M+H]⁺, calculated MW 440.63; **¹H-NMR** (400 MHz, DMSO-*d*₆) d 8.28 (s, 1 H), 7.50 (s, 1 H), 7.44 (t, *J* = 21.4 Hz, 1 H), 7.46 ~ 7.43 (m, 1 H), 7.39 ~ 7.37 (m, 2 H), 6.78 (s, 1 H), 4.95 (s, 2 H).

### Experimental Example 1: Fluorescence-based enzyme activity assay

In order to measure the inhibitory effect of the compounds on the activity of histone deacetylase 6 (HDAC6), an enzyme activity assay was performed to evaluate the enzyme activity by reacting the human HDAC6 enzyme with a substrate to which a fluorescent peptide of p53 residues 379-382 was attached. The test compounds were dissolved in 100% DMSO at a concentration of 10 mM to prepare a stock solution. A starting concentration of 30 µM was prepared from the stock solution, followed by 1/3 serial dilutions to prepare the test buffer solution. Trichostatin A (TSA)-which is the reference compound used to confirm the validity of the experiment-was serially diluted by 1/3 with the test buffer solution, starting from the highest concentration of 1 µM. To react the test compounds and TSA prepared in the test buffer with the HDAC6 enzyme, they were dispensed into a 96-well black plate and spin-down of the plate was performed. Then, the reaction was carried out at room temperature for 10 minutes. After 10 minutes, the substrate was dispensed, and the plate was sealed. The reaction was carried out at 30°C for 1 hour under light-blocking conditions. Fluorescence values were measured using a plate reader (wavelength: excitation/emission = 360/460 nm). The fluorescence measurements were normalized to the HDAC6 enzyme-treated group as 100% and the untreated group as 0%, respectively. The activity of the compounds was calculated as IC₅₀ value and represented in Table 1 (+: 500 - 1,000 nM, ++: 100 - 500 nM, +++: less than 100 nM).

**[Table 1]**

| **Example No.** | **HDAC6 (IC₅₀, nM)** | **Example No.** | **HDAC6 (IC₅₀, nM)** |
|---|---|---|---|
| 1 | +++ | 21 | +++ |
| 2 | + | 22 | +++ |
| 3 | ++ | 23 | +++ |
| 4 | ++ | 24 | +++ |
| 5 | ++ | 25 | ++ |
| 6 | + | 27 | +++ |
| 7 | + | 28 | + |
| 8 | +++ | 29 | +++ |
| 9 | +++ | 30 | + |
| 10 | +++ | 31 | +++ |
| 11 | +++ | 32 | +++ |
| 12 | +++ | 33 | +++ |
| 13 | +++ | 34 | +++ |
| 14 | +++ | 35 | +++ |
| 15 | +++ | 36 | ++ |
| 16 | +++ | 40 | ++ |
| 17 | ++ | 41 | + |
| 18 | +++ | 42 | +++ |
| 19 | ++ | 43 | +++ |
| 20 | ++ | 45 | ++ |

### Experimental Example 2: Fluorescence-based Ca²⁺ mobilization assay

In order to measure the activity of the antagonist on the mGluR5, an experiment was carried out to check the change in the intracellular Ca²⁺ level using the HEK293 cell line permanently overexpressing the mGluR5. The cells in the cell culture medium were aliquoted into a 384-well plate coated with poly-D-lysine and cultured in a 37°C incubator supplied with 5% CO₂. The next day, the medium was removed and a dye loading buffer-in which a reagent capable of measuring Ca²⁺ was dissolved-was added, and then incubated at 37°C for 60 minutes. The test compounds were dissolved in 100% DMSO at a final concentration of 10 mM to make a stock solution. The starting concentration of 10 µM was prepared from the stock solution, followed by serial dilutions to prepare working solutions (test compound working solutions). The test compound working solutions were added to the cells that were incubated in the dye loading buffer, which were maintained for 30 minutes at room temperature in a light-shielding state before measurement. The Ca²⁺ level change induced by L-glutamate at EC₈₀ concentration was measured for 2 minutes using FLIPR Tetra (MDS Analytical Technologies). The fluorescent Ca²⁺ signal values in the presence of the test compounds were normalized to the fluorescent signal elicited by EC₈₀ concentration of L-glutamate as 100% and that elicited by vehicle as 0% to estimate % inhibition of the test compounds. The antagonistic efficacy of the test compounds was calculated as IC₅₀ values and represented in Table 2 (+: 1,000-2,000 nM, ++: 500-1,000 nM, +++: 100-500 nM, ++++: less than 100 nM).

**[Table 2]**

| **Example No.** | **Human mGluR5 (IC₅₀, nM)** | **Example No.** | **Human mGluR5 (IC₅₀, nM)** |
|---|---|---|---|
| 7 | + | 28 | + |
| 15 | ++ | 30 | ++ |
| 17 | + | 31 | ++ |
| 18 | + | 36 | + |
| 19 | + | 37 | ++ |
| 20 | + | 38 | ++ |
| 23 | + | 39 | ++ |
| 24 | ++ | 44 | + |
| 25 | ++ | 45 | ++ |
| 26 | ++ | 46 | ++ |
| 27 | + | | |

## Claims

1. A compound of the following Formula 1 or a pharmaceutically acceptable salt thereof: 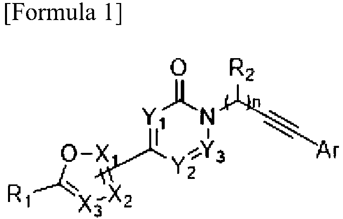 in Formula 1,
Ar is aryl or heteroaryl, wherein the aryl and heteroaryl are optionally substituted with one or more substituents selected from the group consisting of halo, cyano, alkyl, haloalkyl, alkoxy, haloalkoxy and alkylsulfonyl;
R₁ is alkyl or haloalkyl;
R₂ is H or alkyl;
one of X₁, X₂ and X₃ is C, and the others are N;
Y₁, Y₂ and Y₃ are each independently CR₃ or N; wherein R₃ is H, halo or alkyl;
n is an integer from 0 to 2; and
the heteroaryl has one or more heteroatoms selected from N, O and S.

2. The compound or pharmaceutically acceptable salt thereof according to Claim 1, wherein
Ar is C₆-C₁₀ aryl or 5- to 10-membered heteroaryl, wherein the aryl and heteroaryl are optionally substituted with one or more substituents selected from the group consisting of halo, cyano, C₁-C₇ alkyl, halo-C₁-C₇ alkyl, C₁-C₇ alkoxy, halo-C₁-C₇ alkoxy and C₁-C₇ alkylsulfonyl;
R₁ is C₁-C₇ alkyl or halo-C₁-C₇ alkyl;
R₂ is H or C₁-C₇ alkyl;
one of X₁, X₂ and X₃ is C, and the others are N;
Y₁, Y₂ and Y₃ are each independently CR₃ or N; wherein R₃ is H, halo or C₁-C₇ alkyl;
n is an integer of 1 or 2; and
the heteroaryl has 1 to 3 heteroatoms selected from N, O and S.

3. The compound or pharmaceutically acceptable salt thereof according to Claim 1, wherein the compound of Formula 1 is a compound of the following Formula 2: 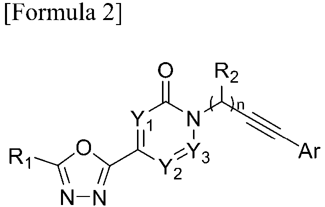 in Formula 2,
n is an integer of 1 or 2; and Ar, R₁, R₂, Y₁, Y₂ and Y₃ are the same as defined in Claim 1.

4. The compound or pharmaceutically acceptable salt thereof according to Claim 1, wherein the compound of Formula 1 is a compound of the following Formula 3: 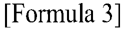 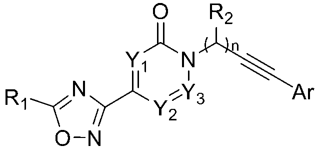 in Formula 3,
n is an integer of 1 or 2; and Ar, R₁, R₂, Y₁, Y₂ and Y₃ are the same as defined in Claim 1.

5. The compound or pharmaceutically acceptable salt thereof according to Claim 1, wherein the aryl is phenyl; and the heteroaryl is pyridyl, thienyl, imidazolyl, or benzothiazolyl.

6. The compound or pharmaceutically acceptable salt thereof according to Claim 1, wherein all of Y₁, Y₂ and Y₃ are CR₃.

7. The compound or pharmaceutically acceptable salt thereof according to Claim 6, wherein R₃ is H.

8. The compound or pharmaceutically acceptable salt thereof according to Claim 1, wherein the compound of Formula 1 is selected from the group consisting of:
4-(5-(difluoromethyl)-1,3,4-oxadiazol-2-yl)-1-(3-phenylprop-2-yn-1-yl)pyridin-2(1H)-one;
1-(3-phenylprop-2-yn-1-yl)-4-(5-(trifluoromethyl)-1,2,4-oxadiazol-3-yl)pyridin-2(1H)-one;
1-(3-(pyridin-4-yl)prop-2-yn-1-yl)-4-(5-(trifluoromethyl)-1,2,4-oxadiazol-3-yl)pyridin-2(1H)-one;
1-(3-(pyridin-3-yl)prop-2-yn-1-yl)-4-(5-(trifluoromethyl)-1,2,4-oxadiazol-3-yl)pyridin-2(1H)-one;
1-(3-(pyridin-2-yl)prop-2-yn-1-yl)-4-(5-(trifluoromethyl)-1,2,4-oxadiazol-3-yl)pyridin-2(1H)-one;
1-(3-(2-fluorophenyl)prop-2-yn-1-yl)-4-(5-(trifluoromethyl)-1,2,4-oxadiazol-3-yl)pyridin-2(1H)-one;
1-(3-(3-chlorophenyl)prop-2-yn-1-yl)-4-(5-(trifluoromethyl)-1,2,4-oxadiazol-3-yl)pyridine-2(1H)-one;
4-(5-(difluoromethyl)-1,3,4-oxadiazol-2-yl)-1-(3-(pyridin-4-yl)prop-2-yn-1-yl)pyridin-2(1H)-one;
4-(5-(difluoromethyl)-1,3,4-oxadiazol-2-yl)-1-(3-(pyridin-3-yl)prop-2-yn-1-yl)pyridin-2(1H)-one;
4-(5-(difluoromethyl)-1,3,4-oxadiazol-2-yl)-1-(3-(pyridin-2-yl)prop-2-yn-1-yl)pyridin-2(1H)-one;
4-(5-(difluoromethyl)-1,3,4-oxadiazol-2-yl)-1-(3-(4-fluorophenyl)prop-2-yn-1-yl)pyridin-2(1H)-one;
4-(5-(difluoromethyl)-1,3,4-oxadiazol-2-yl)-1-(3-(3-fluorophenyl)prop-2-yn-1-yl)pyridin-2(1H)-one;
4-(5-(difluoromethyl)-1,3,4-oxadiazol-2-yl)-1-(3-(2-fluorophenyl)prop-2-yn-1-yl)pyridin-2(1H)-one;
1-(3-(4-chlorophenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(difluoromethyl)-1,3,4-oxadiazol-2-yl)pyridin-2(1H)-one;
1-(3-(2-chlorophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(p-tolyl)prop-2-yn-1-yl)pyridin-2(1H)-one;
4-(5-(difluoromethyl)-1,3,4-oxadiazol-2-yl)-1-(3-(m-tolyl)prop-2-yn-1-yl)pyridin-2(1H)-one;
4-(5-(difluoromethyl)-1,3,4-oxadiazol-2-yl)-1-(3-(3-(trifluoromethyl)phenyl)prop-2-yn-1-yl)pyridin-2(1H)-one;
4-(5-(difluoromethyl)-1,3,4-oxadiazol-2-yl)-1-(3-(3-(trifluoromethoxy)phenyl)prop-2-yn- 1-yl)pyridin-2(1H)-one;
4-(5-(difluoromethyl)-1,3,4-oxadiazol-2-yl)-1-(3-(3-methoxyphenyl)prop-2-yn-1-yl)pyridin-2(1H)-one;
1-(3-(6-chloropyridin-2-yl)prop-2-yn-1-yl)-4-(5-(difluoromethyl)-1,3,4-oxadiazol-2-yl)pyridin-2(1H)-one;
1-(3-(2-chloropyridin-4-yl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(6-methylpyridin-2-yl)prop-2-yn-1-yl)pyridin-2(1H)-one;
1-(3-(3-bromophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(3-(difluoromethyl)phenyl)prop-2-yn-1-yl)pyridin-2(1H)-one;
1-(3-(3-(difluoromethoxy)phenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(trifluoromethyl)-1,3,4-oxadiazol-2-yl)pyridin-2(1H)-one;
4-(5-(difluoromethyl)-1,3,4-oxadiazol-2-yl)-1-(3-(5-methylpyridin-3-yl)prop-2-yn-1-yl)pyridin-2(1H)-one;
1-(3-(3-chlorophenyl)prop-2-yn-1-yl)-4-(5-(difluoromethyl)-1,2,4-oxadiazol-3-yl)pyridin-2(1H)-one;
3-(3-(4-(5-(difluoromethyl)-1,3,4-oxadiazol-2-yl)-2-oxopyridin-1(2H)-yl)prop-1-yn-1-yl)benzonitrile;
4-(5-(difluoromethyl)-1,3,4-oxadiazol-2-yl)-1-(3-(3-(methylsulfonyl)phenyl)prop-2-yn-1-yl)pyridin-2(1H)-one;
1-(3-(3-chloro-2-fluorophenyl)prop-2-yn-1-yl)-4-(5-(difluoromethyl)-1,3,4-oxadiazol-2-yl)pyridin-2(1H)-one;
1-(3-(3-chloro-4-fluorophenyl)prop-2-yn-1-yl)-4-(5-(difluoromethyl)-1,3,4-oxadiazol-2-yl)pyridin-2(1H)-one;
1-(3-(3-chloro-5-fluorophenyl)prop-2-yn-1-yl)-4-(5-(difluoromethyl)-1,3,4-oxadiazol-2-yl)pyridin-2(1H)-one;
1-(3-(5-chloro-2-fluorophenyl)prop-2-yn-1-yl)-4-(5-(difluoromethyl)-1,3,4-oxadiazol-2-yl)pyridin-2(1H)-one;
2-(3-(3-chlorophenyl)prop-2-yn-1-yl)-5-(5-(difluoromethyl)-1,3,4-oxadiazol-2-yl)pyridazin-3(2H)-one;
1-(3-(3-chlorophenyl)prop-2-yn-1-yl)-4-(5-(difluoromethyl)-1,3,4-oxadiazol-2-yl)pyrimidin-2(1H)-one;
3-(3-(3-chlorophenyl)prop-2-yn-1-yl)-6-(5-(difluoromethyl)-1,3,4-oxadiazol-2-yl)pyrimidin-4(3H)-one;
1-(3-(benzo[*d*]thiazol-5-yl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(2-methylbenzo[d]thiazol-6-yl)prop-2-yn-1-yl)pyridin-2(1H)-one;
4-(5-(difluoromethyl)-1,3,4-oxadiazol-2-yl)-1-(3-(2-methyl-1H-imidazol-5-yl)prop-2-yn-1-yl)pyridin-2(1H)-one;
4-(5-(difluoromethyl)-1,3,4-oxadiazol-2-yl)-1-(3-(thiophen-3-yl)prop-2-yn-1-yl)pyridin-2(1H)-one;
1-(1-(3-chlorophenyl)pent-1-yn-3-yl)-4-(5-(difluoromethyl)-1,3,4-oxadiazol-2-yl)pyridin-2(1H)-one;
1-(3-(3-chlorophenyl)prop-2-yn-1-yl)-4-(5-(difluoromethyl)-1,3,4-oxadiazol-2-yl)-6-methylpyridin-2(1H)-one; and
5-bromo-1-(3-(3-chlorophenyl)prop-2-yn-1-yl)-4-(5-(difluoromethyl)-1,3,4-oxadiazol-2-yl)pyridin-2(1H)-one.

9. The compound or pharmaceutically acceptable salt thereof according to Claim 8, wherein the compound of Formula 1 is selected from the group consisting of:
1-(3-(3-chlorophenyl)prop-2-yn-1-yl)-4-(5-(trifluoromethyl)-1,2,4-oxadiazol-3-yl)pyridin-2(1H)-one;
1-(3-(3-chlorophenyl)prop-2-yn-1-yl)-4-(5-(difluoromethyl)-1,3,4-oxadiazol-2-yl)pyridin-2(1H)-one;
4-(5-(difluoromethyl)-1,3,4-oxadiazol-2-yl)-1-(3-(p-tolyl)prop-2-yn-1-yl)pyridin-2(1H)-one;
4-(5-(difluoromethyl)-1,3,4-oxadiazol-2-yl)-1-(3-(m-tolyl)prop-2-yn-1-yl)pyridin-2(1H)-one;
4-(5-(difluoromethyl)-1,3,4-oxadiazol-2-yl)-1-(3-(3-(trifluoromethyl)phenyl)prop-2-yn-1-yl)pyridin-2(1H)-one;
4-(5-(difluoromethyl)-1,3,4-oxadiazol-2-yl)-1-(3-(3-(trifluoromethoxy)phenyl)prop-2-yn-1-yl)pyridin-2(1H)-one;
1-(3-(2-chloropyridin-4-yl)prop-2-yn-1-yl)-4-(5-(difluoromethyl)-1,3,4-oxadiazol-2-yl)pyridin-2(1 H)-one;
4-(5-(difluoromethyl)-1,3,4-oxadiazol-2-yl)-1-(3-(6-methylpyridin-2-yl)prop-2-yn-1-yl)pyridin-2(1H)-one;
1-(3-(3-bromophenyl)prop-2-yn-1-yl)-4-(5-(difluoromethyl)-1,3,4-oxadiazol-2-yl)pyridin-2(1H)-one;
1-(3-(3-(difluoromethoxy)phenyl)prop-2-yn-1-yl)-4-(5-(difluoromethyl)-1,3,4-oxadiazol-2-yl)pyridin-2(1H)-one;
1-(3-(3-chlorophenyl)prop-2-yn-1-yl)-4-(5-(trifluoromethyl)-1,3,4-oxadiazol-2-yl)pyridin-2(1H)-one;
1-(3-(3-chlorophenyl)prop-2-yn-1-yl)-4-(5-(difluoromethyl)-1,2,4-oxadiazol-3-yl)pyridin-2(1H)-one;
3-(3-(4-(5-(difluoromethyl)-1,3,4-oxadiazol-2-yl)-2-oxopyridin-1(2H)-yl)prop-1-yn-1-yl)benzonitrile;
1-(3-(5-chloro-2-fluorophenyl)prop-2-yn-1-yl)-4-(5-(difluoromethyl)-1,3,4-oxadiazol-2-yl)pyridin-2(1H)-one;
1-(1-(3-chlorophenyl)pent-1-yn-3-yl)-4-(5-(difluoromethyl)-1,3,4-oxadiazol-2-yl)pyridin-2(1H)-one; and
1-(3-(3-chlorophenyl)prop-2-yn-1-yl)-4-(5-(difluoromethyl)-1,3,4-oxadiazol-2-yl)-6-methylpyridin-2(1H)-one.

10. A pharmaceutical composition for the prevention or treatment of movement disorder comprising a therapeutically effective amount of the compound or pharmaceutically acceptable salt thereof as defined in any one of Claims 1 to 9 as active ingredient, together with a pharmaceutically acceptable carrier or excipient.

11. The pharmaceutical composition according to Claim 10, wherein the movement disorder is Huntington's disease, Parkinson's disease or levodopa-induced dyskinesia (LID).
